# EUROPEAN PATENT APPLICATION

(11) **EP 1 542 016 A1**
(43) Date of publication of application: **15.06.2005**
(21) Application number: 03293115.6
(22) Date of filing: 11.12.2003
(51) Int. Cl.: G01N 33/569

(54) **PUMA, phosphorylated p53 and p38 map kinase as markers for HIV-1-induced apoptosis in circulating T cells of infected patient**

(71) Applicant: Institut Gustave Roussy, F-94800 Villejuif (FR); INSTITUT NATIONAL DE LA SANTE ET DE LA RECHERCHE MEDICALE (INSERM), 75013 Paris (FR); Université Paris Sud, 91400 Orsay (FR)
(72) Inventor: Kroemer, Guido, 92160 Antony (FR); Perfettini, Jean-Luc, 75012 Paris (FR); Piacentini, Mauro, 00184 Roma (IT)
(74) Representative: Le Forestier, Eric

(57) **Abstract**

The subject matter of the present invention relates to an *in vitro* method for the screening of anti-HIV compounds, or for predicting the progression of HIV related diseases, or for determining the efficiency of an anti-HIV-1 treatment based on the measuring of the expression level of Puma and, optionally, on the measuring of the phosphorylated p53 and/or the p38 MAP kinase protein level in circulating white blood cells of a sample from a HIV infected patient, particularly HIV-1. The invention further comprises a method for the diagnostic of HIV infection in a patient and kits for performing these methods.

SEQUENCE LISTING

<110> Institut Gustave-Roussy
      Institut National de la Santé et de la Recherche Médicale
      (INSERM)
      Université Paris-Sud

<120> Puma, phosphorylated p53 and p38 MAP kinase as markers for
      HIV-1-induced apoptosis in circulating T cells of infected
      patient

<130> D21741

<160> 12

<170> PatentIn version 3.2

<210> 1
<211> 21
<212> DNA
<213> Artificial

<220>
<221> source
<222> (1)..(21)
<223> /note="description of artificial sequence: Forward primer derived
       from Human bcl-2 gene"

<400> 1
catgtgtgtg gagagcgtca a                                                21


<210> 2
<211> 17
<212> DNA
<213> Artificial

<220>
<221> source
<222> (1)..(17)
<223> /note="description of artificial sequence: Reverse primer derived
       from Human bcl-2 gene"

<400> 2
caggtgtgca ggtgccg                                                     17


<210> 3
<211> 19
<212> DNA
<213> Artificial

<220>
<221> source
<222> (1)..(19)
<223> /note="description of artificial sequence: Forward primer derived
       from Human bax-a gene"

<400> 3
ccaaggtgcc ggaactgat                                                   19

<210> 4
<211> 21
<212> DNA
<213> Artificial

<220>
<221> source
<222> (1)..(21)
<223> /note="description of artificial sequence: Reverse primer derived
      from Human bax-a gene"

<400> 4
aagtaggaga ggaggccgtc c                                                21


<210> 5
<211> 19
<212> DNA
<213> Artificial

<220>
<221> source
<222> (1)..(19)
<223> /note="description of artificial sequence: Forward primer derived
      from Human bax-β gene"

<400> 5
ccaaggtgcc ggaactgat                                                   19


<210> 6
<211> 21
<212> DNA
<213> Artificial

<220>
<221> source
<222> (1)..(21)
<223> /note="description of artificial sequence: Reverse primer derived
      from Human bax-β gene"

<400> 6
gaggaggctt gaggagtctc a                                                21


<210> 7
<211> 23
<212> DNA
<213> Artificial

<220>
<221> source
<222> (1)..(23)
<223> /note="description of artificial sequence: Forward primer derived
      from Human bak gene"

<400> 7
accgacgcka tgactcagag ttc                                              23

<210> 8
<211> 18
<212> DNA
<213> Artificial

<220>
<221> source
<222> (1)..(18)
<223> /note="description of artificial sequence: Reverse primer derived
      from Human bak gene"

<400> 8
acacggcacc aattgatg                                                    18


<210> 9
<211> 18
<212> DNA
<213> Artificial

<220>
<221> source
<222> (1)..(18)
<223> /note="description of artificial sequence: anti-sense
      oligonucleotide derived from Human puma gene"

<400> 9
catccttctc atactttc                                                    18


<210> 10
<211> 18
<212> DNA
<213> Artificial

<220>
<221> source
<222> (1)..(18)
<223> /note="description of artificial sequence: Control anti-sense
      oligonucleotide"

<400> 10
ctttcatact cttcctac                                                    18


<210> 11
<211> 21
<212> DNA
<213> Artificial

<220>
<221> source
<222> (1)..(21)
<223> /note="description of artificial sequence: Doubled-stranded
      morpholino oligonucleotide specific for Human bax gene"

<400> 11
cuggacagua acauggagct t                                                21


<210> 12

<211> 21
<212> DNA
<213> Artificial

<220>
<221> source
<222> (1)..(21)
<223> /note="description of artificial sequence: Doubled-stranded
      morpholino oligonucleotide specific for Human bak gene"

<400> 12
uggucccauc cugaacgugt t                                                21

## Description

The subject matter of the present invention relates to an *in vitro* method for the screening of anti-HIV compounds, or for predicting the progression of HIV related diseases, or for determining the efficiency of an anti-HIV-1 treatment based on the measuring of the expression level of Puma and, optionally, on the measuring of the phosphorylated p53 and/or the p38 MAP kinase protein level in circulating white blood cells of a sample from a HIV infected patient, particularly HIV-1. The invention further comprises a method for the diagnostic of HIV infection in a patient and kits for performing these methods.

The acquired immunodeficiency syndrome (AIDS) caused by human immunodeficiency virus (HTV-1) involves the apoptotic destruction of lymphocytes (1-3). The envelope glycoprotein complex (Env) constitutes one of the major apoptosis inducers encoded by the human immunodeficiency virus (HIV-1). The soluble Env derivative gp120 can induce apoptosis through interactions with suitable surface receptors (3). HIV-1 infected cells which express mature Env (the gp120/gp41 complex) on the surface also can kill uninfected cells expressing the receptor (CD4) and the co-receptor (CXCR4 for lymphotropic Env variants, CCR5 for monocytotropic Env variants). This type of bystander killing is obtained by at least two distinct mechanisms. First, the two interacting cells (one which expresses Env and the other that expresses CD4 plus the co-receptor) may not fuse entirely and simply exchange plasma membrane lipids, after a sort of hemi-fusion process, followed by caspase-independent death. Second, the two cells can undergo cytoplasmic fusion (cytogamy), thus forming a syncytium, and then undergo nuclear fusion (karyogamy) and apoptosis (4). In syncytia, the cyclin B-dependent kinase-1 (Cdkl) is activated, a process which is required for karyogamy and which culminates in the mTOR-mediated phosphorylation of p53 on serine 15. The transcriptional activation of p53 then results in the expression of pro-apoptotic proteins including Bax and cell death (5). p53 and Bax have also been found to participate in the death of HIV-1-infected primary lymphoblasts (9). Both the syncytium-dependent and -independent Env-induced apoptosis are suppressed by inhibitors of the gp41- and gp120- mediated (co)-receptor interactions. Moreover, both are reduced by transfection with the Bax antagonist Bcl-2 (4). In contrast, only the syncytium-dependent cell death can be blocked by inhibitors of Cdkl (e.g. roscovitine), mTOR (e.g. rapamycin), or p53 (e.g. cyclic pifithrin-α) (5).

Some of the characteristics of syncytium-induced apoptosis, namely aberrant cyclin B expression (7), overexpression/activation of mTOR (5), phosphorylation of p53 on serine 15 (5, 7), and destabilization of mitochondrial membranes (4), also affect a subset of lymph node and peripheral blood lymphocytes from HIV-1 infected donors, correlating with viral load (5, 7-9). This suggests that the pro-apoptotic signal transduction pathway that can be studied in Env-elicited syncytia reflects - to some extent - the HIV-1-stimulated cell death, as observable among circulating lymphocytes.

It has been observed that the activating phosphorylation of p53 on serine 15 is found in lymphocyte (6) or monocyte (7) cultures infected with HIV-1 *in vitro,* in lymph node biopsies from HIV-1 infected donors (5), as well as peripheral blood mononuclear cells of HIV-1 infected individuals, correlating with viral load (7). It has been also disclosed that transfection with dominant-negative p53 mutants or treatment with a pharmacological p53 inhibitor, cyclic pifithrin-alpha (27), prevents the Env-induced upregulation of Bax and thus retards syncytial cell death (5, 7). Transcriptome analyses performed on HIV-1 infected cultures revealed the induction of p53 target genes including Bax (6, 28).

The activation of the mitochondrial death pathway by p53 involves transcriptional (29) and perhaps non-transcriptional effects (30). The transcriptional activity of p53 and its preferential activation on apoptosis-inducing (rather than cell cycle-arresting) genes depends on a series of post-transcriptional modifications, one of which is phosphorylation of p53 on serine 46 (p53S46P) (20). This activating phosphorylation can be mediated by ATM (presumably in an indirect fashion) and directly by homeodomain-interacting protein (HIP) kinase-2 (32), or by the p38 MAPK (33).

Based on this above-cited prior art, the inventors have explored the proapoptotic signal transduction pathway participating in Env-induced syncytium-dependent cell killing and, to further investigated the implication of p53 in HIV-1 Env-induced apoptosis, have investigated the implication of p53S46P and putative p53S46P kinases in the death process. They have analyzed Env-elicited changes in the transcriptome and established the preponderant role of NF-kB as well as of p53 as pro-apoptotic transcription factors in HIV-1-induced cell death. Moreover, the inventors have identified Puma as a pro-apoptotic, p53-inducible protein which is critical for in Env-induced apoptosis and furthermore have found that p38 MAPK-mediated p53 phosphorylation constitutes a critical step of HIV-1-induced apoptosis.

So, based on the finding of this proapoptotic signal transduction pathway, it is the object of the present invention to provide new method for the screening of anti-HIV compounds, for predicting the progression or for the diagnostic of HIV related diseases, or for determining *in vitro* the efficiency of an anti-HIV treatment.

So, in a first aspect the present invention invention is directed to a method for the *in vitro* screening of anti-HIV compounds comprising the following steps of:
(a) forming a mixture of a compound to be tested with a sample containing HIV infected circulating white blood cells;
(b) measuring of the Puma expression level and/or the phosphorylated p38 MAP kinase protein level and/or the phosphorylated p53 protein level in said HIV infected circulating white blood cells,
wherein a compound that decreases the phosphorylated p38 MAP kinase protein level and/or the phosphorylated p53 protein level and/or the Puma expression level in said circulating white blood cells being selected as an anti-HIV compound.

In a first preferred embodiment, the present invention concerns a method for the *in vitro* screening of anti-HIV compounds according to the invention comprising the following steps of:
(a) forming a mixture of a compound to be tested with a sample containing HIV infected circulating white blood cells;
(b) measuring of the Puma expression level in said HIV infected circulating white blood cells,
wherein a compound that decreases the Puma expression level in said circulating white blood cells is selected as an anti-HIV compound.

It is also preferred in this first aspect a method for the *in vitro* screening of anti-HIV compounds according to the invention wherein in addition to the measuring of the Puma expression level, the step (b) further comprises a step of measuring of the phosphorylated p38 MAP kinase protein level and/or the phosphorylated p53 protein level in said HIV infected circulating white blood cells, and wherein a compound that decreases in said circulating white blood cells:
- the Puma expression level; and
- the phosphorylated p38 MAP kinase protein level and/or (preferably "and") the phosphorylated p53 protein level, is selected as an anti-HIV compound.

By the terms "anti-HIV compound" it is intended to mean compound capable of reducing the white blood cells death induced by HIV infection in a patient or capable of prolongating their survival time. "Anti-HIV compound"also is intended to mean a compound wich results to the diminution of the delay in the appearance of morphological and/or biochemical changes normally associated with apoptosis of the white blood cells, or a diminution of these changes, or a diminution of the number of cells presenting these morphological and/or biochemical changes. Thus, this invention provides the selection of therapeutic compounds capable of increasing the survival time and/or survival rate of a white cell or population of white cells which, in absence of the use of this selected compound, would normally be expected to die. Accordingly, it also provides compositions comprising such selected compounds by the method according to the invention and methods to prevent or treat diseases or pathological conditions associated with unwanted cell death in a subject infected by HIV.

For the purpose of illustration only, examples of suitable circulating white blood cells in the present description are T lymphocytes (T cells) (e.g., TCR+, CD4+ and CD8+ T cells) or mixed lymphocytes cells (MLC) or bone marrow cells.

In a second aspect the present invention invention is directed to a method for *in vitro* predicting the progression of HIV related diseases in a HIV infected patient, said method comprising the following step:
(a) from a sample of said patient containing HIV infected circulating white blood cells, measuring of the Puma expression level and/or the phosphorylated p38 MAP kinase protein level and/or the phosphorylated p53 protein level in said HIV infected circulating white blood cells,
wherein an increase of the Puma expression level and/or the phosphorylated p38 MAP kinase protein level and/or the phosphorylated p53 protein level in said HTV infected circulating white blood cells, compared with reference circulating white blood cells of a HIV non infected patient, is significant of HIV induced circulating white blood cells apoptosis in said infected patient.

In a first preferred embodiment of this second aspect, the present invention concerns a method for *in vitro* predicting the progression of HIV related diseases in a HIV infected patient according to the invention, said method comprising the following step:
(a) from a sample of said patient containing HIV infected circulating white blood cells, measuring of the Puma expression level in said HIV infected circulating white blood cells,
wherein an increase of the the Puma expression level in said HIV infected circulating white blood cells, compared with reference circulating white blood cells of a HIV non infected patient is significant of HIV induced circulating white blood cells apoptosis in said infected patient.

In a second preferred embodiment of this second aspect, the present invention concerns a method for *in vitro* predicting the progression of HIV related diseases in a HIV infected patient according to the invention, wherein in addition to the measuring of the Puma expression level, the step (a) further comprises a step of measuring of the phosphorylated p38 MAP kinase protein level and/or (preferably "and") the phosphorylated p53 protein level in said HIV infected circulating white blood cells, and wherein an increase in said circulating white blood cells of:
- the Puma expression level; and
- the phosphorylated p38 MAP kinase protein level and/or (preferably "and") the phosphorylated p53 protein level,
when compared with reference circulating white blood cells of a HIV non infected patient, is significant of HIV induced circulating white blood cells apoptosis in said infected patient.

In a third aspect the present invention invention is directed to a method for determining *in vitro* the efficiency of an anti-HIV treatment for a HIV infected and treated patient, said method comprising the following step:
(a) obtaining a sample of said anti-HIV treated patient, said sample containing circulating white blood cells; and
(b) measuring of the Puma expression level and/or the phosphorylated p38 MAP kinase protein level and/or the phosphorylated p53 protein level in said circulating white blood cells,
wherein the efficiency of said anti-HIV treatment is correlated with the decrease of the amount of the Puma expression level and/or the phosphorylated p38 MAP kinase protein level and/or the phosphorylated p53 protein level measured in said circulating white blood cells.

In a forth aspect the present invention invention is directed to a method for determining *in vitro* the efficiency of an anti-HIV treatment susceptible to be administrated to a HIV infected patient, said method comprising the following step:
(a) forming a mixture of the anti-HIV treatment compounds susceptible to be administrated with a sample containing HIV infected circulating white blood cells from a sample of said non-treated patient containing HIV infected circulating white blood cells; and
(b) measuring of the Puma expression level and/or the phosphorylated p38 MAP kinase protein level and/or the phosphorylated p53 protein level in said circulating white blood cells,
wherein the efficiency of said anti-HIV treatment is correlated with the decrease of the amount of the measuring of the Puma expression level and/or the amoun of the phosphorylated p38 MAP kinase protein level and/or the amount of the phosphorylated p53 protein level measured in said circulating white blood cells.

In a first preferred embodiment of these third and forth aspect, the present invention concerns a method for determining *in vitro* the efficiency of an anti-HIV treatment according to the invention, wherein step (b) is a step of measuring of the Puma expression level in said circulating white blood cells, and the efficiency of said anti-HIV treatment is correlated with the decrease of the amount of the measuring of the Puma expression level measured in said circulating white blood cells.

In a second preferred embodiment of these third and forth aspects, the present invention concerns a method for determining *in vitro* the efficiency of an anti-HIV treatment according to the invention, wherein in addition to the measuring of the Puma expression level, the step (b) further comprises a step of measuring of the phosphorylated p38 MAP kinase protein level and/or (preferably "and") the phosphorylated p53 protein level in said HIV infected circulating white blood cells, and wherein the efficiency of said anti-HIV treatment is correlated with the decrease of the amount of:
- the Puma expression level; and
- the phosphorylated p38 MAP kinase protein level and/or (preferably "and") the phosphorylated p53 protein level.

In a fifth aspect the present invention invention is directed to a method for diagnosing *in vitro* the HIV infection of a patient susceptible to be infected with HIV, said method comprising the following step of:
(a) from a sample of said patient susceptible to be infected with HIV, said sample containing circulating white blood cells, measuring of the Puma expression level and/or the phosphorylated p38 MAP kinase protein level and/or the phosphorylated p53 protein level in said circulating white blood cells,
wherein the increase of the amount of the Puma expression level and/or the phosphorylated p38 MAP kinase protein level and/or the phosphorylated p53 protein level measured in said circulating white blood cells, is significant with a HIV infection of said patient.

In a first preferred embodiment of this fifth aspect, the present invention concerns a method for diagnosing *in vitro* the HIV infection according to the invention, wherein step (b) is a step of measuring of the Puma expression level in said circulating white blood cells, and
wherein the increase of the amount of Puma expression level measured in said circulating white blood cells is significant with a HIV infection of said patient.

In a second preferred embodiment of this fifth aspect, the present invention concerns a method for diagnosing *in vitro* the HIV infection according to the invention, wherein in addition to the measuring of the Puma expression level, the step (a) further comprises a step of measuring of the phosphorylated p38 MAP kinase protein level and/or (preferably "and") the phosphorylated p53 protein level in said HIV infected circulating white blood cells, and
wherein the increase measured in said circulating white blood cells of:
- the amount of Puma expression level; and
- the amount of phosphorylated p38 MAP kinase protein level and/or (preferably "and") the phosphorylated p53 protein level, is significant with a HIV infection of said patient.

In the method according to the present invention, it is preferred that the expression level of Puma which is measured in step (a) or (b) is the measuring of the Puma protein level. The methods of the present invention wherein the expression level of Puma level is determined by the measuring of the Puma transcripts level, mRNA or cDNA encoding the Puma protein, forms also part of the present invention.

In the method of the present invention where it is concerned, it is preferred that the measuring of the phosphorylated p53 protein level in step (a) or (b) is the measuring of the phosphorylated p53S46P protein level.

In the method of the present invention where it is concerned, it is also preferred that the measuring of the phosphorylated p38 MAP kinase protein level in step (a) or (b) is the measuring of the phosphorylated p38T180P or/and p38Y182P protein level.

In a preferred embodiment and where it is concerned the measuring of said Puma protein level, said phosphorylated p38 MAP kinase protein level and phosphorylated p53 protein level is carried out by specific antibodies capable of specifically recognizing said proteins.

In a further preferred embodiment, the invention is directed to the method for the screening of anti-HIV compounds, or for predicting the progression of HIV related diseases, or for determining the efficiency of an anti-HIV or for diagnosing HIV infection according to the present invention wherein HIV is HIV-1.

In a sixth aspect the present invention invention is directed to a kit for the *in vitro* diagnostic of HIV infection, preferably HIV-1 infection, in a patient, characterized in that it comprises:
(a) anti-human Puma protein antibodies or a set or primers capable of amplifying the human Puma mRNA.
   The term "primer" denotes a specific oligonucleotide sequence which is complementary to a target nucleotide sequence and used to hybridize to the target nucleotide sequence. A primer serves as an initiation point for nucleotide polymerization catalyzed by DNA polymerase, RNA polymerase or reverse transcriptase.
   In a preferred embodiment, said kit according to the invention further comprises:
(b) anti-human phosphorylated p38 MAP kinase antibodies; and/or
(c) antibodies anti-human phosphorylated p53 protein antibodies.

In the kit of the present invention and where it is concerned:
- the anti-human phosphorylated p38 MAP kinase antibodies are anti-human phosphorylated p38T180P antibodies or/and anti-human phosphorylated p38Y182P antibodies; and/or
- the anti-human phosphorylated p53 protein antibodies are anti-human phosphorylated p53S46P antibodies.

The term "antibody" as used herein refers to immunoglobulin molecules and immunologically active portions of immunoglobulin molecules, i.e., molecules that contain an antigen binding site which specifically binds (immunoreacts with) the human Puma protein, such as the peptide depicted in GenBank NM 014417, or the phosphorylated p38T180P or/and p38Y182P protein, such as the peptide depicted in GenBank NM 002751 having that or these phosphorylated amino acids, or the phosphorylated p53S46P protein, such as the peptide depicted in GenBank AY 429684 having that phosphorylated amino acid, or against a specific fragment thereof.

The term "antibody" as used herein which specifically binds the phosphorylated p38T180P or/and p38Y182P protein or the phosphorylated p53S46P protein, refers to a population of antibody molecules that bind to that part of particular phosphorylated p38 or p53 protein, but do not bind to the non-phosphorylated p38 or p53 protein or to the p38 or p53 protein phosphorylated in an other point of the sequence, so that they permit to identify and to quantify specifically the presence of said particular phosphorylated p38 or p53 proteins.

The term "antibody" comprises monoclonal or polyclonal antibodies.

An isolated human Puma protein, such as the peptide depicted in GenBank NM 014417, or phosphorylated p38T180P or p38Y182P protein, such as the peptide depicted in GenBank NM 002751 having that or these phosphorylated amino acids, or phosphorylated p53S46P protein, such as the peptide depicted in GenBank AY 429684 having that phosphorylated amino acid, or a a specific fragment thereof can be used as an immunogen to generate antibodies that bind such protein using standard techniques for polyclonal and monoclonal antibody preparation. It may be also possible to use any fragment of these protein which contains at least one antigenic determinant, particularly containing the phosphorylated aminoacid for p53 and p38, may be used to generate these specific antibodies.

A protein immunogen typically is used to prepare antibodies by immunizing a suitable subject, (e.g., rabbit, goat, mouse or other mammal) with the immunogen. An appropriate immunogenic preparation can contain said protein, or fragment thereof, and further can include an adjuvant, such as Freund's complete or incomplete adjuvant, or similar immunostimulatory agent.

Thus, antibody for use in accordance with the invention include either polyclonal or monoclonal antibodies capable of selectively binding, or which selectively bind to an epitope-containing a polypeptide comprising a contiguous span of at least 8 to 10 amino acids of an amino acid sequence of the protein, including the phosphorylated aminoacid for p53 and p38.

An exemplary method for determining the level of the human Puma protein, or the phosphorylated p38T180P or/and p38Y182P protein, or the phosphorylated p53S46P protein is depicted in the examples below. The determination of the level of the human Puma protein, or nucleic acid encoding said human Puma protein, or the phosphorylated p38T180P or p38Y182P protein, or the phosphorylated p53S46P protein in a biological sample involves obtaining a biological sample from a test subject and contacting the biological sample with a compound or an agent capable of detecting their level.

A preferred agent for detecting and quantifying mRNA or cDNA encoding said human Puma protein, is a labeled nucleic acid probe or primers capable of hybridizing this mRNA or cDNA. The nucleic acid probe can be an oligonucleotide of at least 10, 15, 30, 50 or 100 nucleotides in length and sufficient to specifically hybridize under stringent conditions to the mRNA or cDNA. The nucleic acid primer can be an oligonucleotide of at least 10, 15 or 20 nucleotides in length and sufficient to specifically hybridize under stringent conditions to the mRNA or cDNA, or complementary sequence thereof.

A preferred agent for detecting and quantifying the the human Puma protein, or the phosphorylated p38T180P or/and p38Y182P protein, or the phosphorylated p53S46P protein is an antibody capable of binding specifically to this protein, preferably an antibody with a detectable label. Antibodies can be polyclonal, or more preferably, monoclonal. An intact antibody, or a fragment thereof (e.g., Fab or F(ab')2) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin.

For example, *in vitro* techniques for detection of candidate mRNA include Northern hybridizations and in situ hybridizations. In vitro techniques for detection of the candidate protein include enzyme linked immunosorbent assays (ELISAs), Western blots, immunoprecipitations and immunofluorescence. In vitro techniques for detection of candidate cDNA include Southern hybridizations.

In one embodiment, the biological sample contains protein molecules from the test subject. Alternatively, when Puma transcripts have to be quantifyied, the biological sample can contain mRNA molecules from the test subject or cDNA molecules from the test subject.

In another embodiment, the methods further involve obtaining a control biological sample from a control subject, contacting the control sample with the agent capable of determining the level of the expression of the human Puma or the phosphorylated human p38T180P or/and p38Y182P protein, or the phosphorylated human p53S46P protein such that the level of these proteins is determined in the control biological sample, and comparing the level of these proteins in the control sample with their level in the test sample.

When the invention encompasses kits for quantifying the level of these proteins, the kit can comprise a labeled compound or agent capable of quantifying these proteins. Said agents can be packaged in a suitable container. The kit can further comprise instructions for using the kit to quantify the level of these proteins or of the Puma transcrips.

In certain embodiments, the determination of the Puma transcripts alteration involves the use of a probe/primer in a polymerase chain reaction (PCR), such as anchor PCR or RACE PCR, or, alternatively, in a ligation chain reaction (LCR) (see, e.g., Landegran et al. (1988) Science 241:23-1080; and Nakazawa et al. (1994) Proc. Natl. Acad. Sci. USA 91:360-364). This method can include the steps of collecting a sample of cells from a patient, isolating nucleic acid (e.g. mRNA) from the cells of the sample, contacting the nucleic acid sample with one or more primers which specifically hybridize to the mRNA under conditions such that hybridization and amplification of the mRNA occurs, and quantifying the presence of the amplification products. It is anticipated that PCR and/or LCR may be desirable to use as an amplification step in conjunction with any of the techniques used for quantifying nucleic acid detecting.

The methods described herein may be performed, for example, by utilizing prepackaged diagnostic kits comprising at least one probe nucleic acid or set of primer or antibody reagent described herein, which may be conveniently used, e.g., in clinical settings to follow-up or diagnose patients.

Finally, the present invention is related to the use of antisense or RNAi (RNA interference) oligonucleotides specific of the nucleic acid encoding human PUMA protein for the manufacture of a medicament intented to prevent or to treat HIV-induced apoptosis, of circulating white blood cells in a HIV infected patient, particularly HIV-1 infected patient.

RNA interference (RNAi) is a phenomenon in which a double stranded RNA (dsRNA) specifically suppresses the expression of a gene bearing its complementary sequence. The phenomenon has been originally discovered in Caenorhabditis elegans by Fire and Mello (35). RNAi has since become a useful research tool for many organisms. Although the mechanism by which dsRNA suppresses gene expression is not entirely understood, experimental data provide important insights. This technology has great potential as a tool to study gene function in mammalian cells and may lead to the development of pharmacological agents based upon siRNA (small interfering RNA) (36, 37, 38).

It is to be understood that while the invention has been described in conjunction with the above embodiments, that the foregoing description and the following examples are intended to illustrate and not limit the scope of the invention. Other aspects, advantages and modifications within the scope of the invention will be apparent to those skilled in the art to which the invention pertains.

### Legends to figures

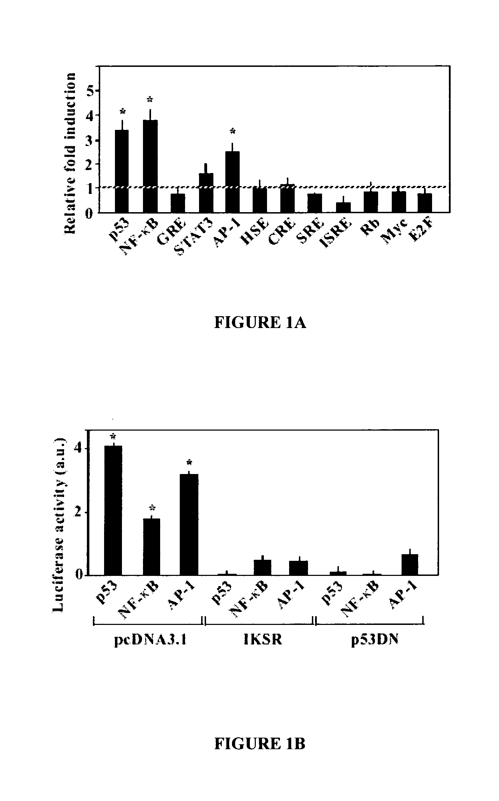

### Figures 1A and 1B: Transcription factor profiling of syncytia elicited by HIV-Env.

Figure 1A. Systematic analysis of transcription factors activated in syncytia. HeLa CD4 cells were transfected with luciferase constructs placed under the control of the indicated transcription factor. Twenty-four hours after transfection, cells were cultured alone (100% control value) or in the presence of HeLa Env cells, and the expression of luciferase was monitored 24 h later. Asterisks indicate a significant (p<0.01, Student t test) increase in the luciferase activity (X±SD, n=3).

Figure 1B. Mutual relationship among p53 and NF-κB. Cells were co-transfected with luciferase expression plasmids controlled by p53, NF-κB or AP-1 plus empty vector (pcDNA3.1) or the IκB superrepressor (IKSR) or a dominant-negative p53 (p53H273), and the increase in luciferase activities in syncytia (versus single cells) was quantified. Results are representative of three independent experiments. Asterisks indicate significant induction of luciferase constructs.

### Figures 2A to 2E: Phosphorylation of IκBα and p53 in Env-elicited syncytia.

Figures 2A, 2B and 2C. Representative immunofluorescence microphotographs of pre-karyogamic and karyogamic syncytia (36 hours) with antibodies specific for IκBαS32/36P (figure 2A), p53S15P (figure 2A, 2B), p53S46P (figure 2C), or cytochrome c (figure 2B, figure 2C). The frequency of the indicated phosphorylation events was determined both among single cells (SC) and karyogamic (KG) or pre-karyogamic syncytia.

Figure 2D. Immunoblot confirmation of the phosphorylation of IκBαS32/36P, p53S 15P, and p53S46P in syncytia. The immunodetection of GAPDH was performed to control equal loading.

Figure 2E. Kinetic analysis of phosphorylation events. Syncytia (obtained by coculture of HeLa Env and HeLa CD4 cells during the indicated time points) were subjected to immunostainings as above and the percentage of cells exhibiting the indicated parameters was quantified among the total population of syncytia.

### Figures 3A to 3E: Rules governing the activation of p53 in Env-elicited syncytia.

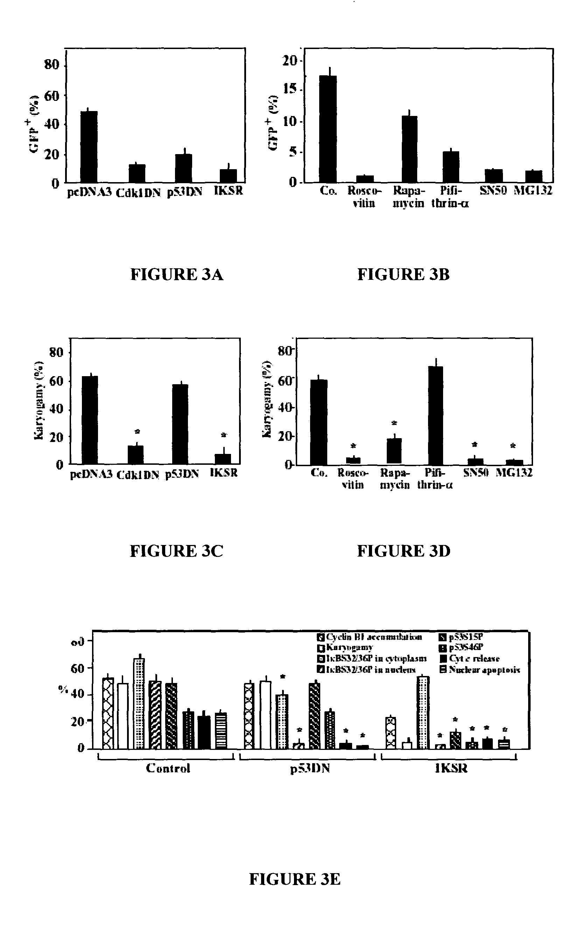

Figure 3A. Genetic inhibition of p53-dependent transcription. HeLa CD4 cells were co-transfected with a p53- inducible GFP construct, together with vector only (pcDNA3.1), a dominant-negative (DN) Cdkl mutant, DN p53 (p53H273), or IKSR. After 24 hours, the cells were either left alone (single cells) or co-cultured with HeLa Env cells for 36 hours, followed by cytofluorometric determination of GFP.

Figure 3B. Pharmacological inhibition of p53-dependent transcription. HeLa CD4 cells were transfected with a p53-inducible GFP construct. After 24 hours, cells were cultured in the presence of the indicated agents, in the presence or absence of HeLa Env cells for 24 hours, followed by determination of the frequency of GFP-expression single cells or syncytia.

Figure 3C. Inhibition of karyogamy by IKSR. HeLa CD4 cells transfected with the indicated constructs as in A and the frequency of cells exhibiting nuclear fusion was scored upon coculture with HeLa Env cells.

Figure 3D. Inhibition of karyogamy by NF-kB inhibitors. Syncytia were treated with various drugs as in B, and the frequency of karyogamic cells was scored.

Figure 3E. Effect of IKSR and DN p53 on cyclin B1, karyogamy, phosphorylation of IkBα or p53, and apoptotic parameters. Syncytia were generated as in A, after transfection with pcDNA3.1 (control, Co.), IKSR, or DN p53, followed by immunofluorescent detection of cyclin B 1. The frequency of syncytia exhibiting an increase in cyclin B1 immunostaining was determined in three independent experiments, after 24 h of coculture. Moreover, the frequency of syncytia exhibiting positive cytoplasmic or nuclear IkBS32/36P staining, p53S15P or p53S46P, mitochondrial cytochrome c release, or nuclear apoptosis was determined. Asterisks indicate significant inhibitory effects (p<0.01, paired student t test, X±SD, n=3).

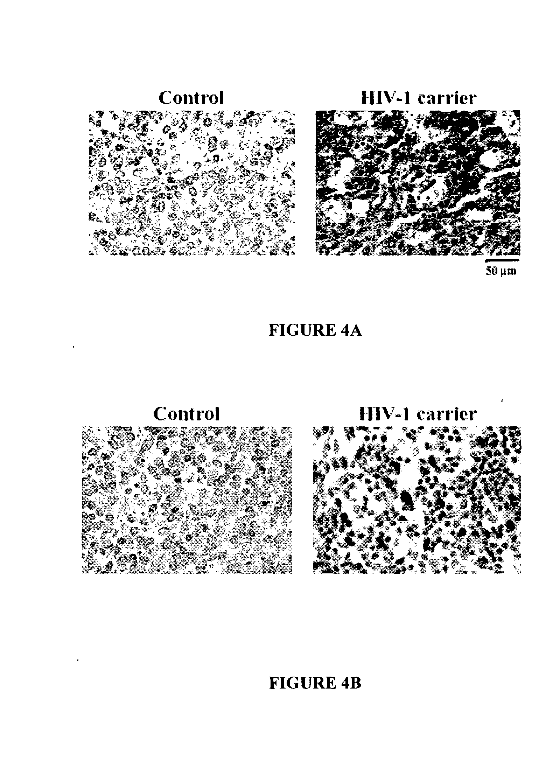

### Figures 4A and 4B: Phosphorylation of IκBα and p53 in lymph nodes from HIV-1 carriers.

Lymph nodes from age- and sex-matched controls or untreated HTV-1 carriers were stained for the immunocytochemical detection (brown) of p53S46P (figure 4A) or IκBαS32/36P (figure 4B). Nuclei are counterstained in blue. Similar results indicating a scattered p53S46 staining and staining of mitotic cells with IκBαS32/36P were found in three independent asymptomatic, untreated HTV-1 infected donors with a viremia of >100 000 copies/ml.

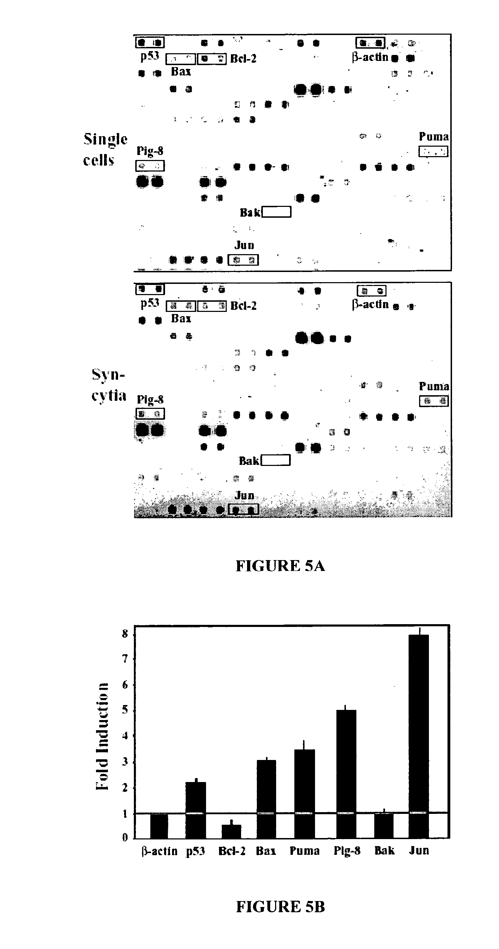

### Figures 5A and 5B: Identification of p53 target genes by macroarray.

Labelled cDNA from single cells or syncytia was hybridized to filters containing p53 target genes. The signal/background ratio was determined for all spots (with ß-actin as an internal control) (figure 5A) and the change in mRNA expression was determined as detailed in Materials and Methods (figure 5B).

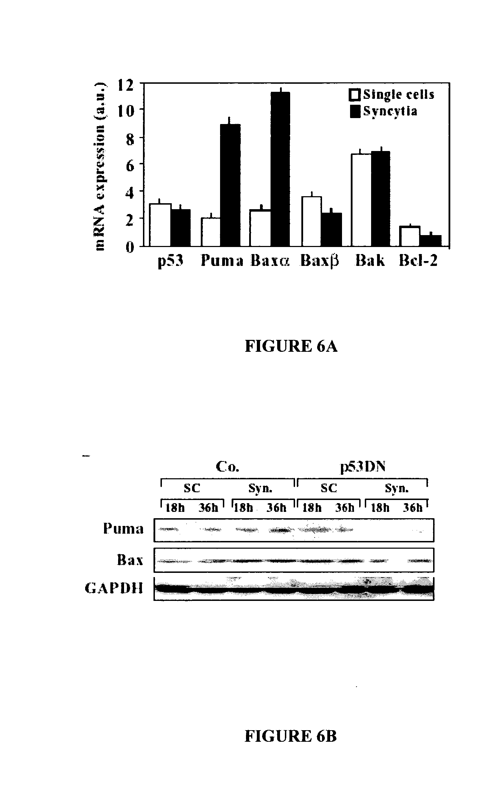

### Figures 6A and 6B: Upregulation of Puma at the mRNA and protein levels.

Figure 6A. Quantitative RT-PCR was used to determine the relative abundance of the indicated transcripts.

Figure 6B. Protein blot detection of Puma expression. Syncytia of different ages (18 or 36 hours of co-culture), generated after transfection with vector only or a p53 DN construct, were subjected the immunodetection of Puma(∼21 kDa), Bax, and GAPDH as a loading control.

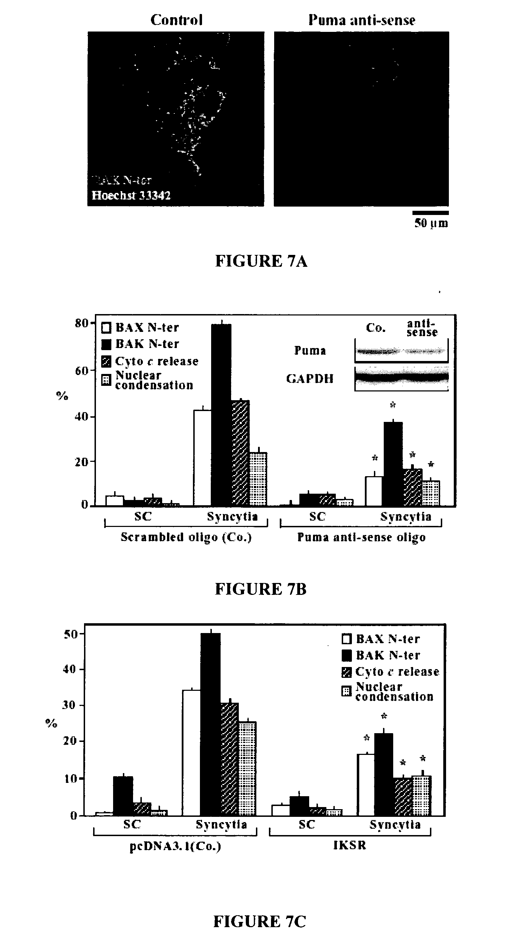

### Figures 7A to 7E: Functional impact of Puma on the Bax/Bak-mediated mitochondrial membrane permeabilization.

Figure 7A. Effect of the Puma anti-sense phosphorothioate oligonucleotide on the conformational change of Bax and Bak. Untreated control syncytia or syncytia generated in the presence of a Puma-specific anti-sense thiorate oligonucleotide were stained with antibodies specific for the N-terminus of Bax (red) or Bak (green) and counterstained with Hoechst 33342. Note that these antibodies only recognize the active, apoptotic conformation of Bax and Bak.

Figure 7B. Quantitation of the effect of the Puma anti-sense oligonucleotide, as compared to a scrambled control oligonucleotide, determined 36 hours after syncytium formation. The insert confirms effective down-regulation of Puma, as determined by immunoblot.

Figure 7C. Effect of IKSR on the activation of Bax. Cells were either transfected with vector only (pcDNA3.1) or with IKSR, 24 hours before the generation of syncytia, and the indicated parameters were assessed in triplicate.

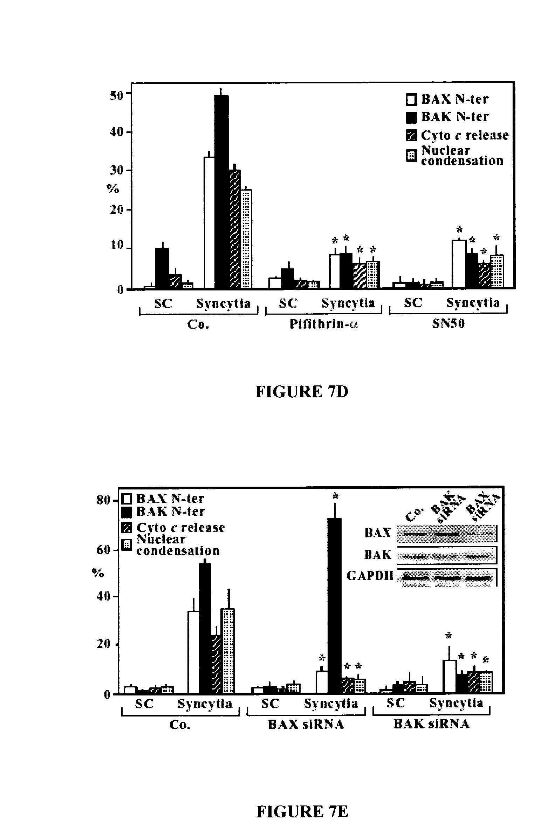

Figure 7D. Effect of SN50 and cyclic pifithrin-alpha as compared to its control (SN50M). The indicated agents were added to HeLa Env and HeLa CD4 at the beginning of co-culture, and the frequency of cells exhibiting activation of Bax or Bak, cytochrome c release and nuclear apoptosis was assessed.

Figure 7E. Effect of Bax and Bak siRNA on the apoptosis of Env-elicited syncytia. Cells were transfected with small interfering RNA oligonucleotides, 24 hours before fusion, and the indicated parameters were assessed. The insert demonstrates the impact of siRNA on Bax and Bak expression, as determined by immunoblot. This experiment has been repeated three times, with similar results.

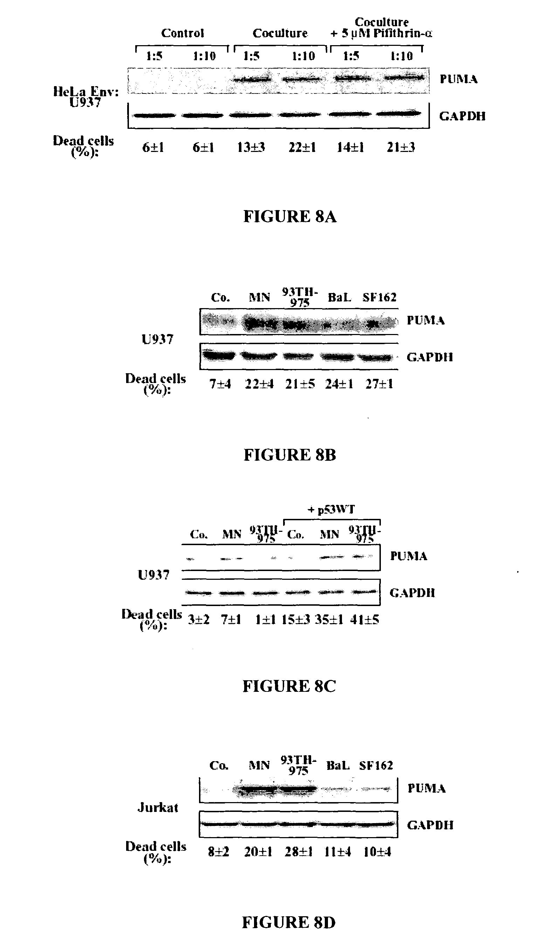

### Figures 8A to 8G: Induction of Puma by Env and HIV-1 in vitro.

Figure 8A. Induction of Puma in U937 cells co-cultured with HeLa Env cells. U937 cells were mixed at different ratios with HeLa Env cells, just before the preparation of cell lysates for immunoblot detection of Puma (Control) or coculture for 36 hours, in the absence or presence of the p53 inhibitor cyclic pifithrin α (5 mM). The percentage of dead U937 cells was determined by trypan blue exclusion.

Figure 8B. Induction of Puma by recombinant gp120 protein in U937 cells. U937 were exposed to the indicated gp120 protein (at 500 ng/ml) and cell death, Puma and GAPDH expression were determined after four days.

Figure 8C. Cooperation between gp120 and p53 to induce Puma in U937 cells. Cells were mock-transfected or transfected with wild type p53. One day later, the indicated gp 120 proteins were added, and the expression of 53 and Puma were determined 48 h later.

Figure 8D. Induction of Puma by recombinant gp120 protein in Jurkat cells (as in figure 8B).

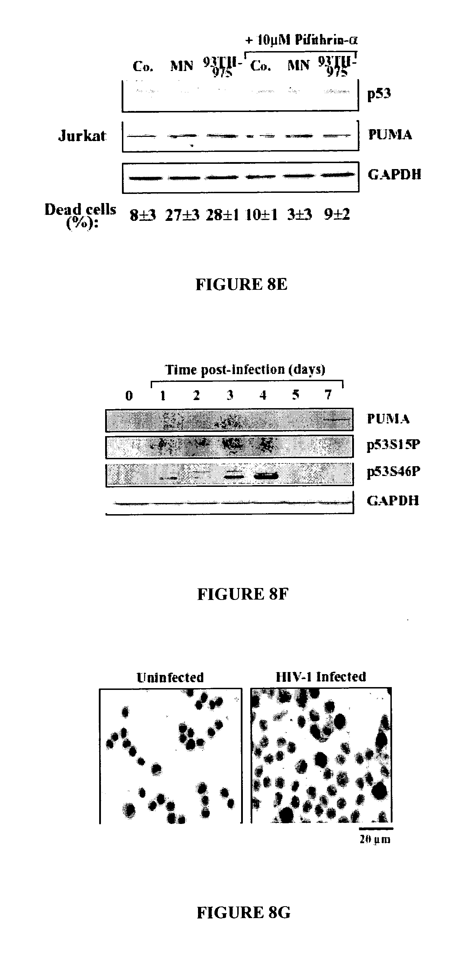

Figure 8E. Effect of cyclic pifithrin-alpha. Jurkat cells were treated for four days with gp120 protein (500 ng/ml) and or cyclic pifithrin-α (10 µM), followed by determination of Puma expression.

Figure 8F. Induction of Puma by infection of primary lymphoblasts *in vitro.* CD4+ lymphoblasts from a healthy donor were infected with HIV-1LAI/IIIb for the indicated period, and proteins (40 µg/lane) were subjected to immunoblot determination of p53 phosphorylation and Puma expression.

Figure 8G. Puma induction in CD4+ lymphoblasts from a healthy donor infected with a clinical HIV-1 isolate. Five days after infection, cells were subjected to immunohistochemical detection of Puma. Uninfected cells served as a negative control. Results typical for five independent experiments are shown.

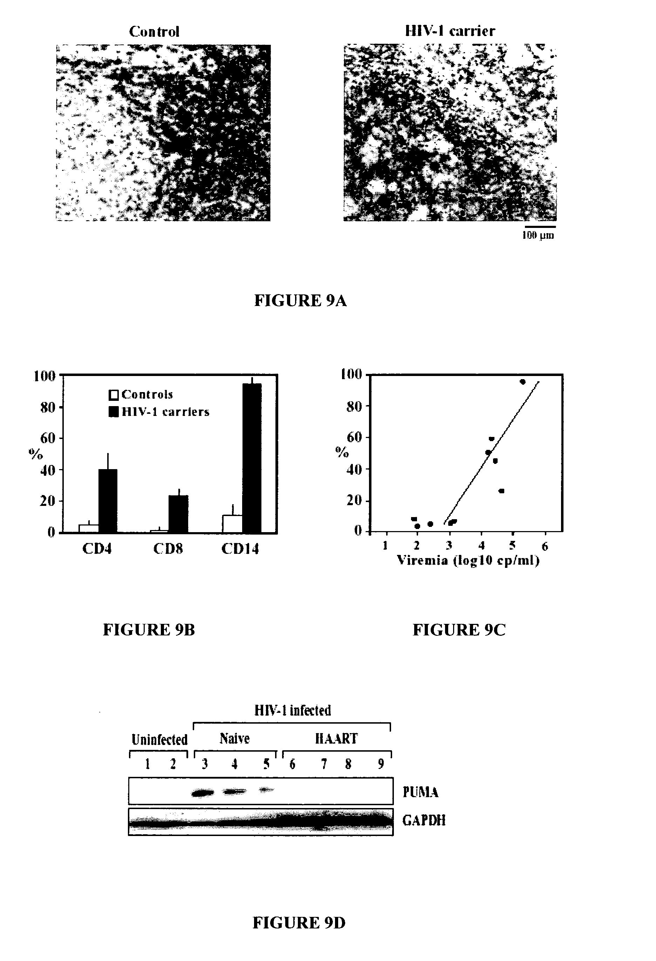

### Figures 9A to 9E: Induction of Puma in HIV-1-infected patients.

Figure 9A. Immunocytochemical localization of Puma in lymph node biopsies of HIV-1 infected donors as compared to a healthy control. Similar results were obtained with three pairs of uninfected and HIV-1 infected donors (> 10⁵ copies per ml).

Figure 9B. Frequency of PUMA+ cells among defined subsets of mononuclear cells. Patients (n=4) and controls (n=4) with the same clinical characteristics as in A were subjected to two color immunofluorescence stainings, allowing for the determination of the percentage (X±SD) of cells expressing Puma among CD4+ or CD8+ lymphocytes, as well among CD 14+ monocytes.

Figure 9C. Correlation between PUMA expression and viral titers. The frequency of PUMA+ cells was determined among total PBMC of 10 untreated HIV-1+ patients. Each dot represents one patient. Calculations were done following the Pearson method.

Figure 9D. Expression of Puma in purified circulating CD4+ lymphocytes from uninfected control donors (Co.), naive HIV-1 infected donors and HIV-1 carriers undergoing successful anti-retroviral therapy (HAART).

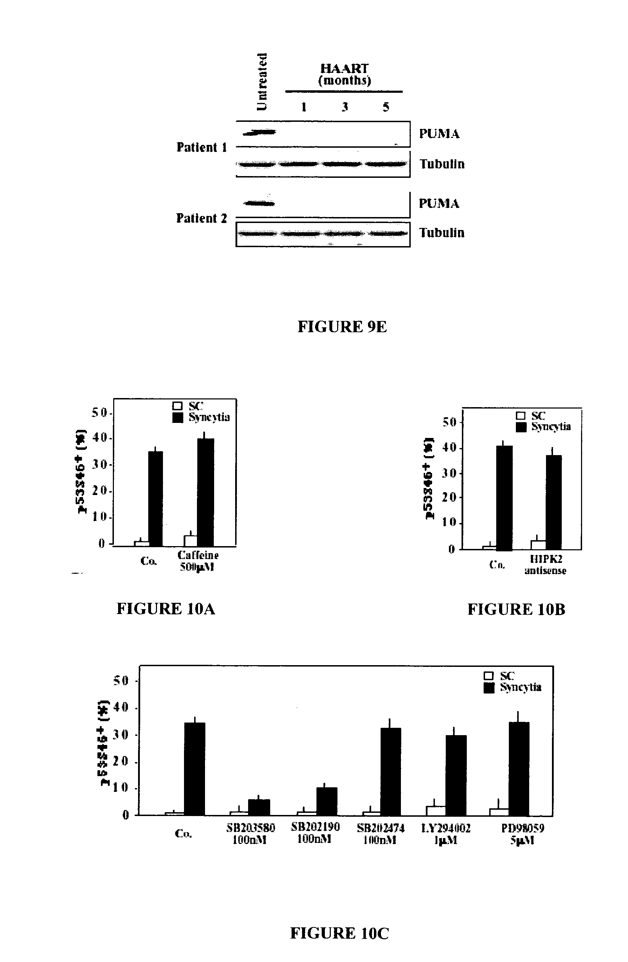

Figure 9E. Longitudinal study of individual patients. Whole PBMC were drawn from freshly diagnosed (untreated) patients, with viral titers > 10⁵ copies/ml, as well as several months after HAART. Note that in these patients, HAART led to a reduction of retroviral titers to levels <5000 within four weeks. Immunoblot detection of Puma and β-tubulin (loading control) is shown.

### Figures 10A to 10C: Inhibition of p53S46 phosphorylation by p38MAPK inhibitors.

Figures 10A and 10C. Inhibition profile of p53S46 phosphorylation. HeLa Env and HeLa CD4 cells were cocultured for 36h in the presence of the indicated agents followed by the determination of the frequency of p53S46P+ syncytia. Results are means of three independent experiments.

Figure 10B. Absence of effect of anti-sense HIPK2 phosphorothioate oligonucleotides on p53S46 phosphorylation in syncytia. Syncytia were generated as in figure 10A, after transfection with anti-sense HIPK2 phosphorothioate oligonucleotides (100 nM) or a scrambled control olignonucleotide (Co.), followed by immunofluorescence detection of p53S46P. The frequency of syncytia exhibiting a positive p53S46P immunostaining was determined in three independent experiments, after 36h of coculture.

### Figures 11A to 11E: Phosphorylation of p38MAPK and ATF-2 in Env-elicited syncytia.

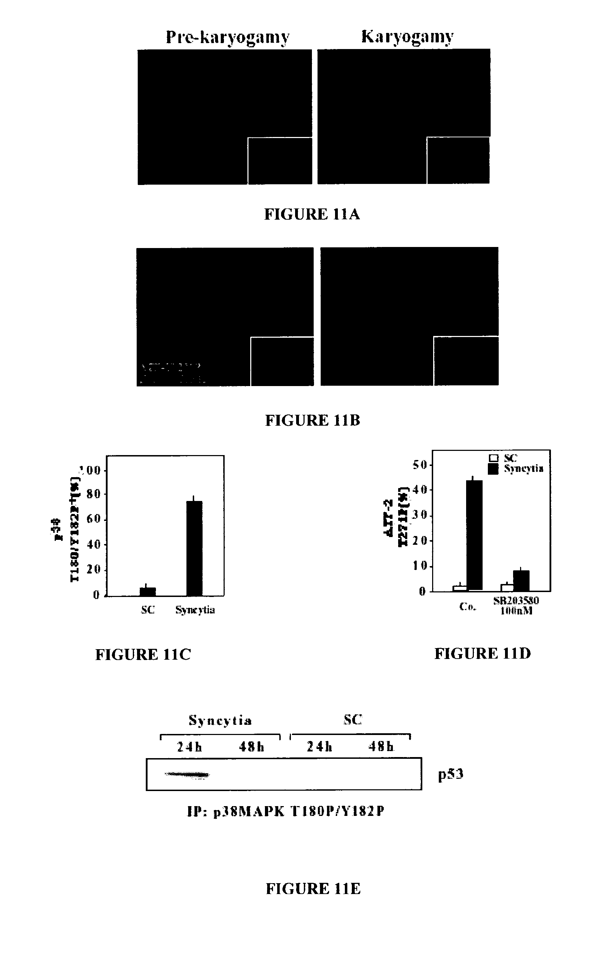

Figures 11A and 11B. Representative immunofluorescence microphotographs of pre-karyogamic and karyogamic syncytia (36 hours) with antibodies specific for p38T180/Y182P (figure 11A) and ATF-2T271P (figure 11B).

Figure 11C. Quantification of p38T180/Y182P in syncytia. The frequency of syncytia exhibiting an increase p38T180/Y182P in immunostaining was determined in three independent experiments, after 36 h of coculture by cytofluorometry.

Figure 11D. Inhibition of ATF-2T271P by SB203580. Syncytia were treated with 100 nM SB203580, and the frequency of cells exhibiting ATF-2T271P immunostaining was scored.

Figure 11E. Co-immunoprecipitation of p38T180/Y182P and p53 in syncytia. Mixtures of HeLa Env and HeLa CD4 SCs, 24-h and 48-h old syncytia were lysed and subjected to immunoprecipitation with rabbit antibody specific for p38T180/Y182P, SDS-PAGE and immunodetection of p53.

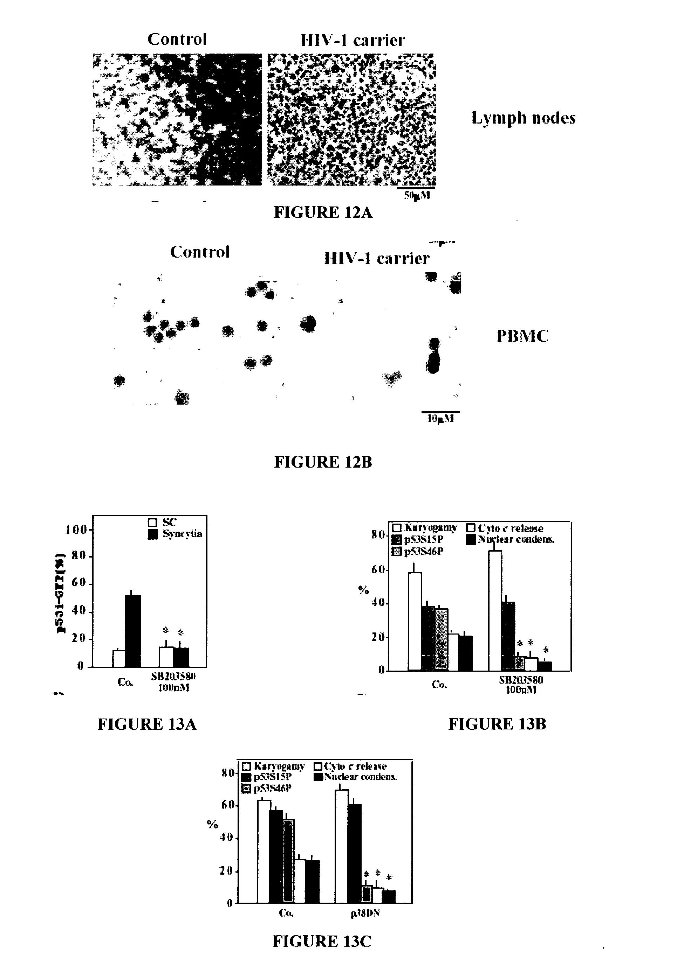

### Figures 12A and 12B: Phosphorylation of p38MAPK in lymph nodes and in PBMC from HIV-1 carriers.

Lymph nodes (figure 12A) and PBMC (figure 12B) from age- and sex-matched controls or untreated HIV-1 carriers were stained for the immunocytochemical detection (red) of p38T180/Y182P (figures 12A and 12B). Nuclei are counterstained in blue. Similar results indicating a p38T180/Y182P staining were found in three independent asymptomatic, untreated HIV-1 infected donors with a viremia of > 10⁵ copies/ml.

### Figures 13A to 13C: Inhibition by p38MAPK inhibitors of p53S46P, p53 transcriptional activity and apoptosis in syncytia.

Figure 13A. Pharmacological inhibition of p53-dependent transcription by SB203580. HeLa CD4 cells were co-transfected with a p53-inducible GFP construct. After 24 hours, the cells were either left alone (single cells) or co-cultured with HeLa Env cells in presence or in absence of SB203580 (100 nM) for 36 hours, followed by cytofluorometric determination of the frequency of GFP-expression single cells or syncytia.

Figure 13B. Pharmacological inhibition of p53S46P and apoptosis in syncytia. Syncytia were treated with 100 nM of SB203580 during 36 h followed by immunofluorescence detection of karyogamy, p53S15P, p53S46P, cytochrome c and nuclear condensation. The frequency of syncytia exhibiting a decrease in p53S46P immunostaining was determined in three independent experiments, after 36 h of coculture. Moreover, the frequency of syncytia exhibiting positive p53S15P, mitochondrial cytochrome c release, or nuclear apoptosis was determined. Asterisks indicate significant inhibitory effects (p<0.01, paired student t test, X±SD, n=3).

Figure 13C. Genetic inhibition of p53S46P and apoptosis in syncytia. Syncytia were generated as in figure 13A, after transfection with pcDNA3.1 (control, Co.), p38 DN, followed by immunofluorescence detection of p53S46P. The frequency of syncytia exhibiting positive p53S 15P or p53S46P, mitochondrial cytochrome c release, or nuclear apoptosis was determined as in figure 13B. Asterisks indicate significant inhibitory effects (p<0.01, paired student t test, X±SD, n=3).

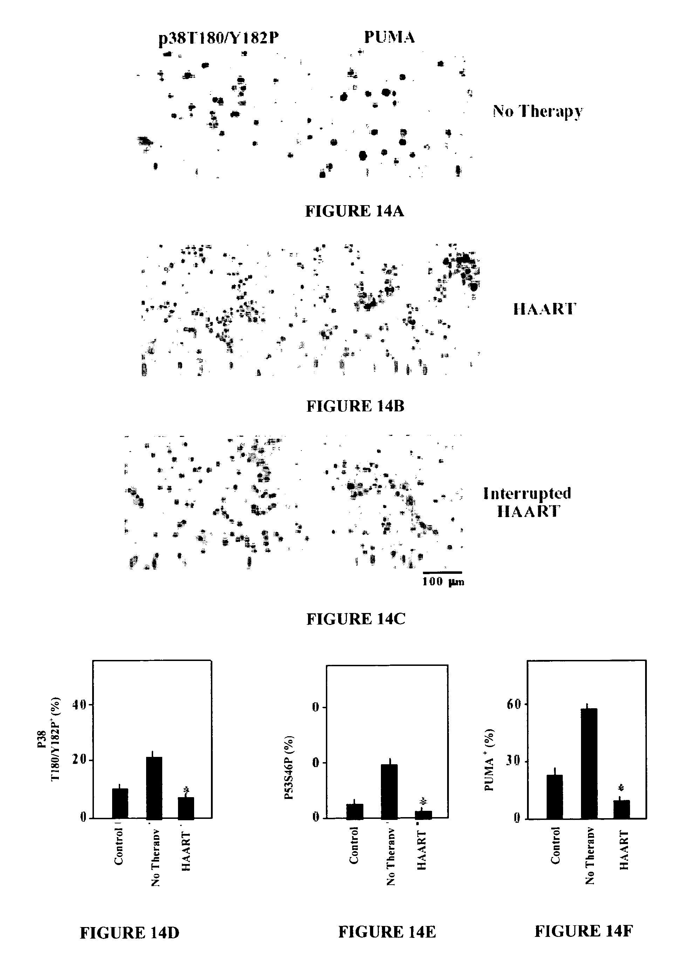

### Figures 14A to 14F: Inhibition by HAART of p38T180/Y182P, p53S46P and PUMA expression.

Figures 14A, 14B and 14C. PBMC from age- and sex-matched untreated, HAART-treated HIV-1 carriers or carriers who interrupted treatment were stained for the immunocytochemical detection (red) of p38T180/Y182P and PUMA. Nuclei are counterstained in blue.

Figures 14D, 14E and 14F. Effect of HAART on p38T180/Y182P, p53S46P and PUMA expression on PBMC from uninfected control donors (figure 14D), naive HIV-1 infected donors (figure 14E) and HIV-1 carriers undergoing successful anti-retroviral therapy (HAART) (figure 14F). Asterisks indicate significant inhibitory effects of HAART as compared with no therapy (p<0.05, paired student t test, X±SD, n=3).

### EXAMPLES

### Example I: NF-κB and p53 are the dominant apoptosis-inducing transcription factors elicited by the HIV-1 envelope

### Abbreviations:

Cdkl, cyclin dependent kinase-1; Cyt. c; cytochrome c; DN, dominant negative; Env, envelope glycoprotein complex; GAPDH, glyceraldehyde-3-phosphate dehydrogenase; HAART, highly active anti-retroviral therapy; HIV, human immunodeficiency virus; IKSR, IκB super-repressor; IκBS32/36P, IκBα phosphorylated on serine 32 and 36; KG, karyogamy; MMP, mitochondrial membrane permeabilization; mTOR, mammalian target of rapamycin; p53S15P, p53 with phosphorylated serine 15; p53S46P, p53 with phosphorylated serine 46; SC, single cell; siRNA, small interfering RNA; Syn, syncytia; Z-VAD.fmk, N-benzyloxycarbonyl-Val-Ala-Asp-fluoromethylketone.

### A) Materials and Methods

### Cells and culture conditions for apoptosis induction

HeLa cells stably transfected with the Env gene of HIV-1 LAI (HeLa Env) and HeLa cells transfected with CD4 (HeLa CD4) were cocultured at a 1:1 ratio (6), in DMEM medium supplemented with 10 % FCS, L-glutamine, in the absence or presence of roscovitin (1 µM), rapamycin (1 µM, Sigma), cyclic pifithrin-α (10 µM), MG132 (5 µM, Calbiochem), the NF-κB-inhibitory peptide SN50, or its negative control SN50M (20 µM, Biomol). HeLa/U937 cell co-cultures were performed at different ratios (6). U937 or Jurkat cells were cultured with recombinant gp120 protein (500 ng/ml).

### Plasmids, transfection, and transcription factor profiling

For transcription factor profiling, HeLa CD4 cells were transfected with different luciferase constructs (MercuryTM pathway profiling system from Clontech, 2 µg DNA) using Lipofectamine 2000TM (In Vitrogen, 2 µl), 24 h before fusion with HeLa Env cells (25 x 10⁴ in 2 ml), and the luciferase activity was measured 24 h later using the luciferase reporter assay kit from ClonTech. Transfection with pcDNA3.1 vector only, wild type (WT) or dominant-negative (DN) CDk1 mutant (14), the IκB super-repressor (IKSR) (gift by Lienhard Schmitz) or p53 DN plasmids (p53H273, gift by Thierry Soussi), or a p53-responsive enhanced green fluorescent (GFP) plasmid (kind gift from Klas Wiman) was performed 24 hours before coculture of HeLa CD4 and HeLa Env cells. The frequency of GFP-expressing cells was assessed by cytofluorometry, on a FACS Vantage (Becton Dickinson) equipped with a 100 µm nozzle, allowing for the analysis of relatively large cells. Cells were after counterstained with Hoechst 33324 (1 µM, 15 min), and the FACS gates were set on syncytia (that is cells with a DNA content >4n) (7).

### Immunofluorescence and cytofluorometry

Rabbit antisera specific for p53S15P, p53S46P (Cell Signaling Technology, MA, USA) or the N-terminus of Bax (N20, Santa Cruz) was used on paraformaldehyde (4% w:v) and picric acid-fixed (0.19 % v:v) cells (15) and revealed with a goat anti-rabbit IgG conjugated to Alexa 568 (red) or Alexa 488 (green) fluorochromes from Molecular Probes. Cells were also stained for the detection of the N-terminus of Bak (Abl, Oncogene) p53S15P (mAb), IκBαS32/36P (mAb, Cell Signaling), revealed by anti-mouse IgG Alexa conjugates. Cells were counterstained with Hoechst 33324 which allows to distinguish karyogamy and apoptotic chromatin condensation (7).

### Immunoblots

Protein samples were prepared from HeLa Env and HeLa CD4 single cells mixed at a 1:1 ratio in lysis buffer (single cells control) or from HeLa Env/CD4 syncytia. Proteins samples from HeLa Env and U937 mixed at different ratios were also prepared and subjected to immunoblot analysis. Aliquots of protein extracts (40 µg) were subjected to immunoblots using antibodies specific for IκBαS32/36P, IκBα, p53S15P, p53S46P, p53 (Cell Signaling Technology), cyclin B1 (mAb, BD Transduction Laboratories), Puma (rabbit anti-human Puma antisera from United States Biological or Orbigen, mouse anti-human Puma antibody from Upstate, which all gave similar results), β-tubulin (mAb from Sigma), or glyceraldehyde-3-phosphated dehydrogenase (GAPDH, mAb from Chemicon).

### Microarrays, macroarrays, and quantitative RT-PCR

HeLa CD4 and HeLa Env cells were labeled with CellTracker™ Red or CellTracker™ Green (15 µM, 30 min at 37°C), respectively, and washed extensively before coculture. After 18 or 36 hours of coculture, in the presence or absence of 10 µM cyclic pifithrin-α (re-added once upon 24 hours), the cells were subjected to FACS purification of syncytia (which are double-positive) or single cells, as described (7). mRNA preparations were obtained with the RNeasy Mini kit (Quiagen), quality-controlled with an Agilent 2100 Bioanalyzer (Agilent Technologies), alternatively labeled with Cy3/Cy5 fluorochromes, mixed for pairwise comparisons, and hybridized to 19 k human arrays. Arrays were scanned with a ScanArray 4000 confocal laser scanner (Perkin Elmer), quantified with QuantArray software (Perkin Elmer) at the Ontario Cancer Institute Microarray Center (http://www.microarrays.ca), and analyzed with GeneTraffic software (Iobion Informatics). All experiments were performed in triplicates, while switching the Cy3/Cy5 labeling (six data points for each pair wise comparison) and upregulations by 50% or downregulations by 30% were listed. Data showing the transcriptional modification of genes with unknown function are listed in Table 1S. Macroarrays were performed using a TranSignal^{TM} p53 target gene array (Panomics, Redwood City, CA), and quantified using the NIH image software. For quantitative RT-PCR, cDNAs were synthesized from 1 µg total RNAs with reverse transcriptase MuLV (Roche). Then, the TaqMan universal PCR was performed on a ABI PRISM® 7000 Sequence Detection System (Applied Biosystems), following the manufacturer's instructions.

The following primer sequences are used for:
- bcl-2: F cat gtg tgt gga gag cgt caa (SEQ ID No. 1), R cag gtg tgc agg tgc cg (SEQ ID No. 2);
- bax α: F cca agg tgc cgg aac tga t (SEQ ID No. 3), R aag tag gag agg agg ccg tcc (SEQ ID No. 4);
- bax β: F cca agg tgc cgg aac tga t (SEQ ID No. 5), R gag gag gct tga gga gtc tca (SEQ ID No. 6);
- bak: F acc gac gck atg act cag agt tc (SEQ ID No. 7), R aca cgg cac caa ttg atg (SEQ ID No. 8); and
- puma: pre-developped Taqman Assays Reagents, www.Appliedbiosystems.com).

### Anti-sense constructs and RNA interference

HPLC-purified anti-sense phosphorothioate oligonucleotides (anti-sense Puma : CAT CCT TCT CAT ACT TTC (SEQ ID No. 9); control: CTT TCA TAG TCT TCC TAC (SEQ ID No. 10), GeneSet Oligos, Paris, France) (100 nM) were transfected with Oligofectamine (Invitrogen) into HeLa CD4 cells, 24 h before co-culture with HeLa Env cells. RNA interference of Bax and Bak expression was obtained by means of double-stranded morpholino oligonucleotides (Genset) specific for Bax (sense strand: 5'-rCUrgrgrArCrArgUrArArCrAUrgrgrArgrCTT-3' (SEQ ID No. 11)) or Bak (sense strand: 5'-UrgrgUrCrCrCrAUrCrCUrgrArArCrgUrgTT-3' (SEQ ID No. 12)), transfected into both HeLa CD4 and HeLa Env cells, 48 h before co-culture.

### Patients' samples and in vitro infection with HIV-1

Axillary lymph node biopsies or peripheral blood samples were obtained from healthy and HIV-1-infected individuals (all males, mean age 36 years), who were naive for HAART with a plasma viral load >20,000 copies/ml; or were receiving HAART with a viral load <5,000 copies/ml. Plasma HIV-1 RNA levels were determined by the bDNA procedure (Versant HIV RNA 3.0) according to the manufacturer's instructions (Bayer, USA). None among the HIV-1+ patients received interferons, glucocorticoids, was positive for hepatitis B or C, or exhibited signs of autoimmunity. PBMCs were isolated by Ficoll/Hypaque (Amersham Pharmacia Biotech) centrifugation of heparinized blood from either healty donors and HIV-seropositive individuals and fixed with 4% paraformaldehyde in PBS pH 7.2. Biopsies were fixed with 10% formalin, dehydrated, and paraffin embedded. Deparaffinized tissue sections and PBMCs were subjected to immunocytochemistry with antibodies specific for p53S46P; IκBαS32/36P or PUMA and counterstained with hematoxylin. PBMC were treated with TRIzol (Invitrogen) to extract total proteins, followed by immunoblot analysis. Alternatively, freshly purified PBMC were stained with anti-CD4 antibody (FITC-labelled OKT3) and FACS-sorted, followed by protein extraction. Primary CD4+ lymphoblasts (10 x 10⁶) cells (5) were incubated with the HIV-1LAI/IIIB strain or different clinical HIV-1 isolates (12) (500 ng of p24) for 4 hours at 37°C. After washing out unabsorbed virus, cells (10⁶ cells/ml) were cultured in RPMI medium containing 20% FCS and 10 U/ml interleukin-2 (IL-2).

### B) Results

### 1- Transcription factor profiling of HIV-1 Env-elicited apoptosis

In a model culture system of syncytium-dependent cell death, HeLa cells stably transfected with the Env gene from HIV-1_{LAI/IIIb} (HeLa Env) were fused by co-culture with CD4/CXCR4-expressing HeLa cells (HeLa CD4) (4, 5). Syncytium-induced transcriptional effects were monitored by transfecting each of the two cell lines with a series of luciferase reporter gene constructs containing promoter elements responsive to a series of different transcription factors. This approach revealed that, among a panel of 12 different transcription factors, only NF-κB, p53, and AP1 were activated by the Env-dependent syncytium formation (Fig. 1A). To determine the hierarchy among these transcription factors, cells were co-transfected with the before mentioned luciferase reporter constructs as well as a non-phosphorylable mutant of IκB (IκB-derived "super-repressor", IKSR) (13) or with a dominant-negative (DN) mutant of p53 (p53H273) (14). Both IKSR and p53H173 abrogated the increase of NF-κB, p53, and AP1 -dependent transcription found in syncytia (Fig. 1B). These data point to an obligate cooperation between NF-κB and p53, upstream of AP1, in Env-elicited syncytia.

### 2- NF-κB is required for Env-elicited mitosis and p53 activation

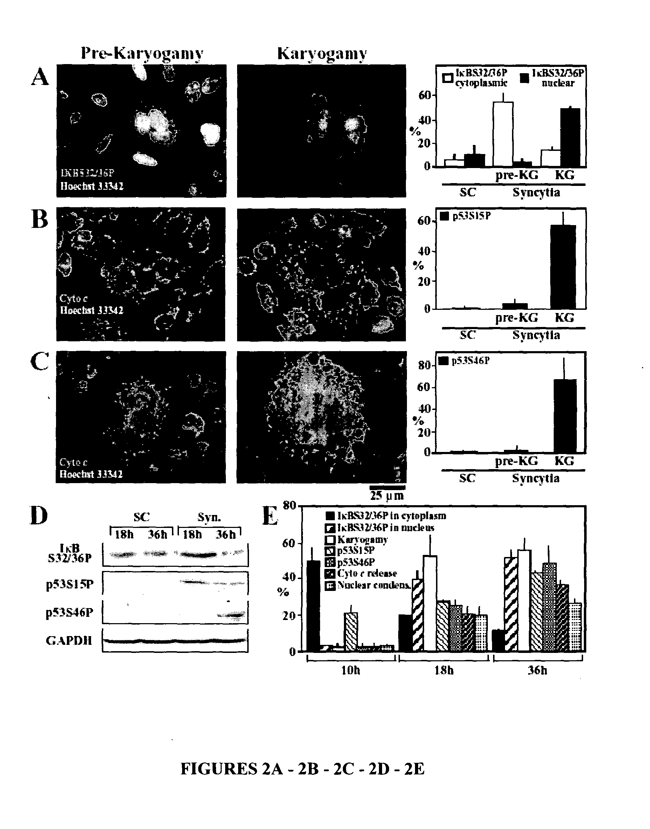

NF-κB is usually considered as an anti-apoptotic transcription factor (15). Intrigued by its putative implication in syncytial apoptosis, we further investigated the possible implication of NF-κB in syncytial apoptosis. Cytoplasmic fusion is followed by nuclear fusion (karyogamy) accompanying an aberrant, cyclin B1-dependent entry into mitosis (7). This allows for the distinction of early (pre-karyogamic) and late (karyogamic) syncytia. A significant fraction of pre-karyogamic syncytia exhibited the phosphorylation of IκB on serine residues 32 and 36 (IκBS32/36P), as detectable with a phosphoneoepitope-specific antibody. IκBS32/36P was found in the cytoplasm of pre-karyogamic syncytia. In karyogamic syncytia, IκBS32/36P was confined to the nucleus (Fig. 2A). p53 was found to exhibit an activation-associated phosphorylation pattern, both on serine 15 (p53S15P) (Fig. 2B) and serine 46 (p53S46P) (Fig. 2C), only in karyogamic syncytia. Immunoblot analysis confirmed the phosphorylation of IKB and p53 (Fig. 2D). Kinetic analysis confirmed a precise order of phosphorylation events (IκBS32/36P --> p53S15P --> p53S46P) (Fig. 2E).

Transfection of HeLa CD4 and HeLa Env cells with a p53-inducible GFP construct allowed for the quantitative assessment of p53-dependent transcriptional effects within syncytia. p53-dependent transcription was strongly inhibited by transfection with IKSR (Fig. 3A), as well as by pharmacological inhibition of the nuclear NF-κB translocation with SN50 (Fig. 3B). Simultaneously, both IKSR (Fig. 3C) and SN50 (Fig. 3D) inhibited the emergence of karyogamic syncytia, suggesting that NF-κB is required for mitotic progression of syncytia. Indeed, NF-κB inhibition fully prevented the syncytial accumulation of cyclin B1, as determined by quantitative immunofluorescence analysis (Fig. 3E). Inhibition of the cyclin B1-dependent kinase-1 (CDK1) by dominant-negative CDK1 (Fig. 3A) or roscovitine (Fig. 3B) inhibited the p53-dependent transcription, while inhibiting karyogamy (Figs. 3C, D). Alltogether, these findings indicate that NF-κB is essential for the aberrant, cyclin B1/Cdk1-mediated entry into karyogamy which occurs upstream of p53 activation. The repression of NF-κB reduced the karyogamy-associated phosphorylation of p53 (both p53S15P and p53S46P). Moreover, transfection with dominant-negative p53 partially inhibited the phosphorylation of IKB and totally blocked its translocation into the nucleus (Fig. 3E). These findings point again to an obligate cross-talk between p53 and NF-κB.

IκB phosphorylation was also detectable in lymph nodes from HIV-1 carriers (but not in those from uninfected controls), mainly among mitotic cells (Fig. 4B). Moreover, the phosphorylation of p53S46P was induced by HN-1 infection of primary CD4+ lymphoblasts (see below) and was detectable in lymph node biopsies from HIV-1 carriers (Fig. 4A). Altogether, these data extend the notion of HIV-1-induced NF-KB activation and p53 activation.

### 3- HTV-1 Env-elicited transcriptional effects involve p53 and lead to Puma overexpression

To further explore the implication of transcription factors in Env-induced syncytial apoptosis, microarrays were performed on HeLa Env/CD4 cocultures maintained for 18 or 36 hours in the presence of absence of cyclic pifithrin-α, a chemical inhibitor of p53. Forty genes of known function (Table I) and 42 genes with unknown function (supplemental Table Is) were found to be significantly (by a factor of = 1.5 or =0.67) induced or inhibited in syncytia, as compared to control cells cultured separately. The chemical inhibitor of p53 transactivation, cyclic pifithrin-α?was found to prevent these changes in transcription, either at 18 or 36 hours, for 85% among these genes, indicating that p53 is indeed the principal transcription factor whose activation directly or indirectly (via effects on other transcription factors) determines the pathophysiology of syncytial apoptosis.

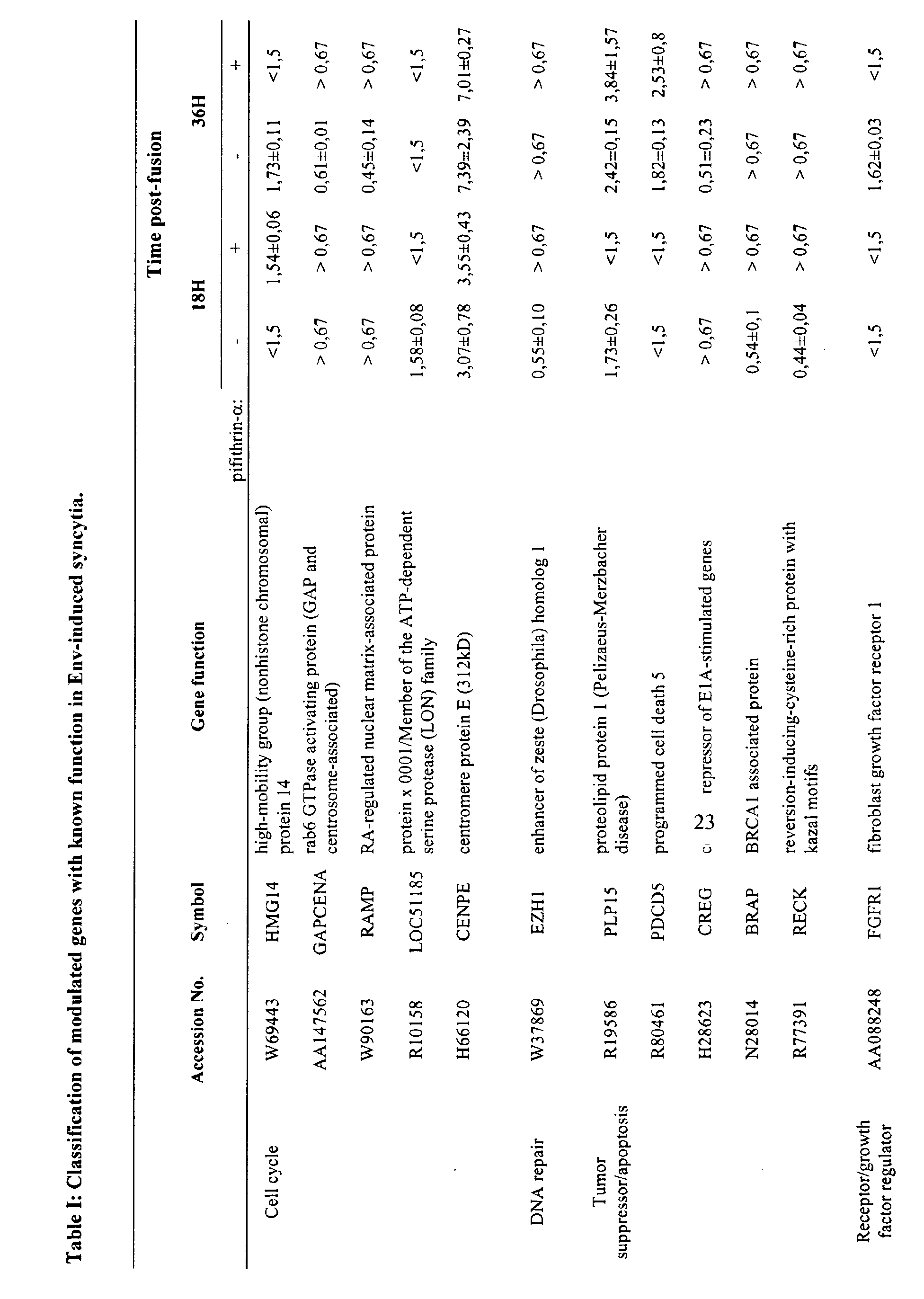

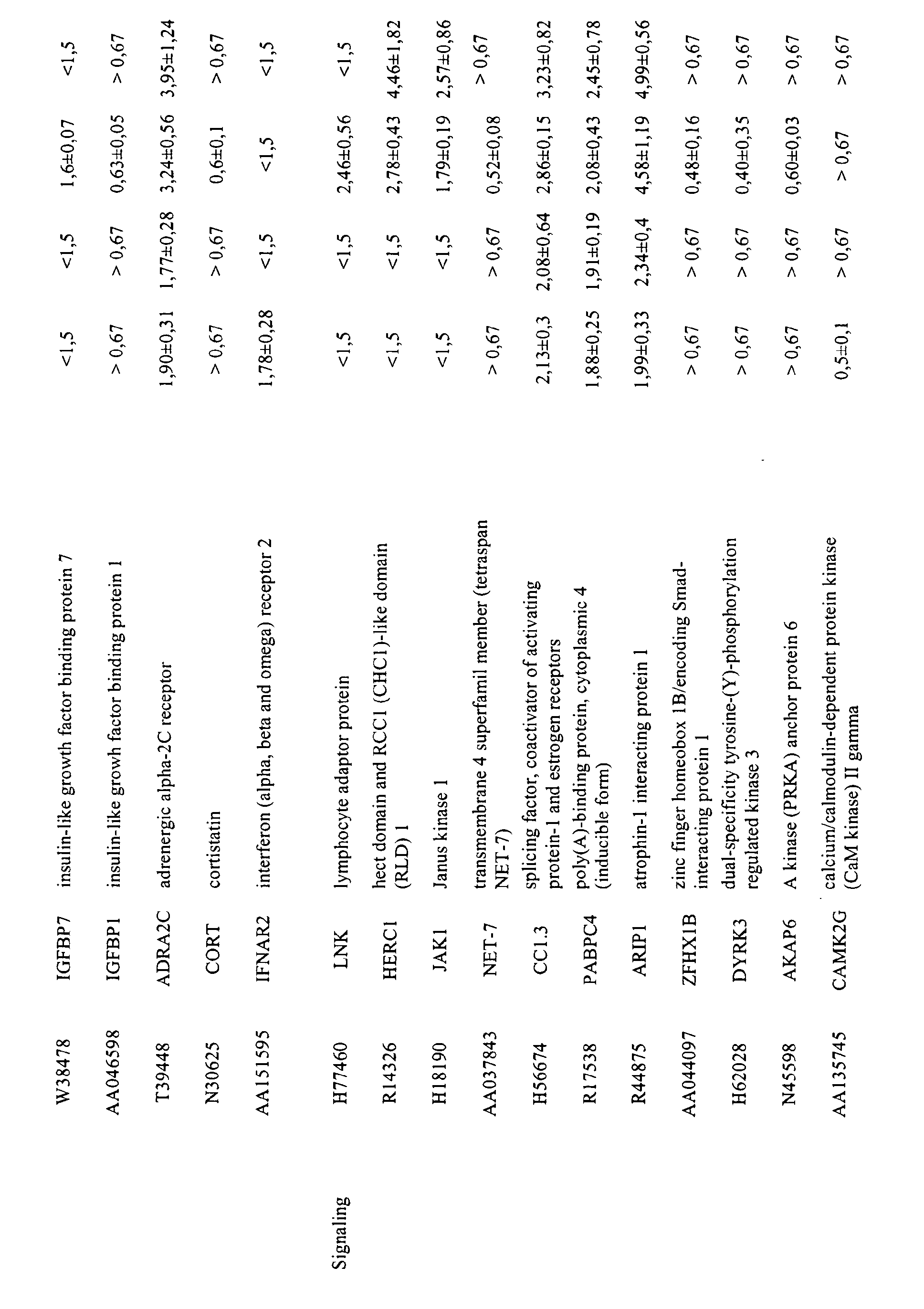

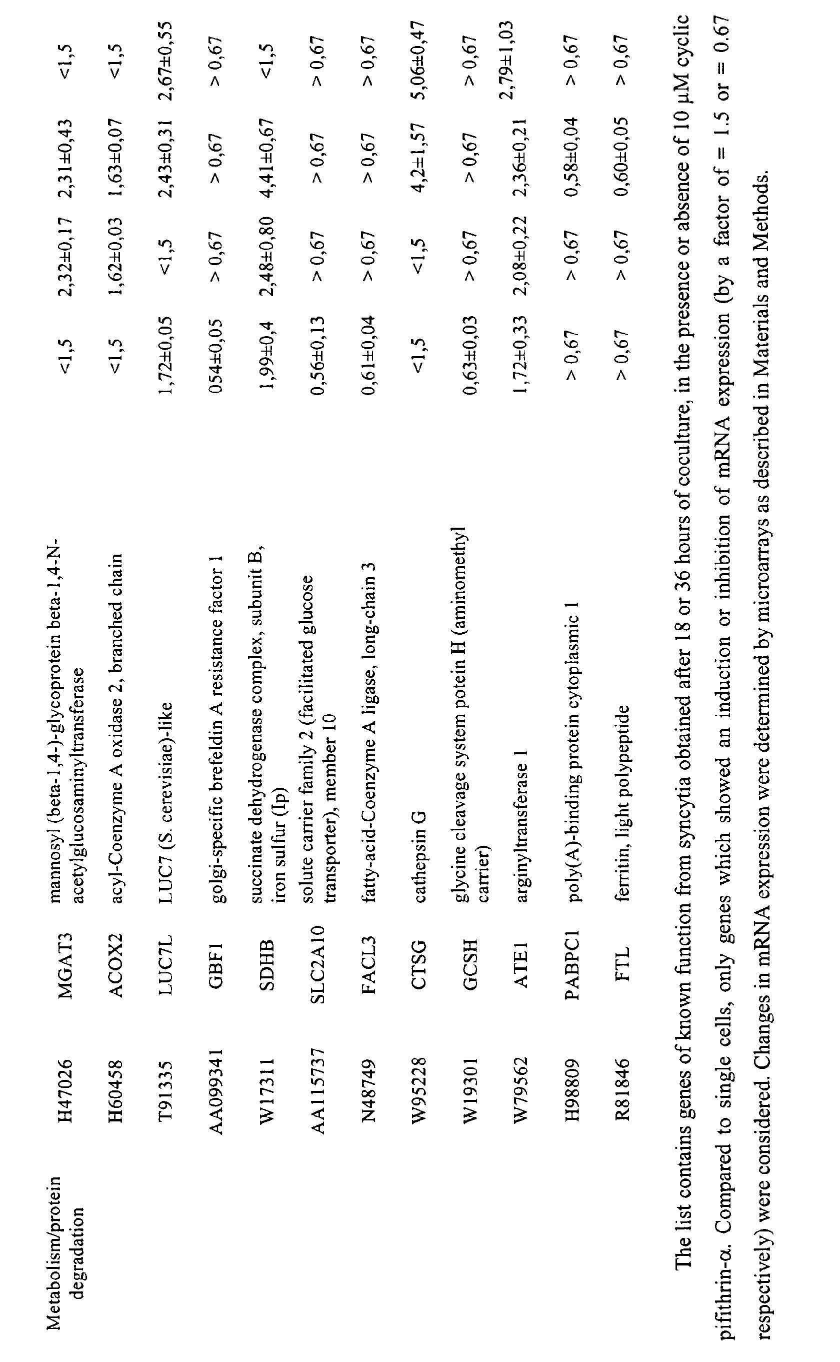

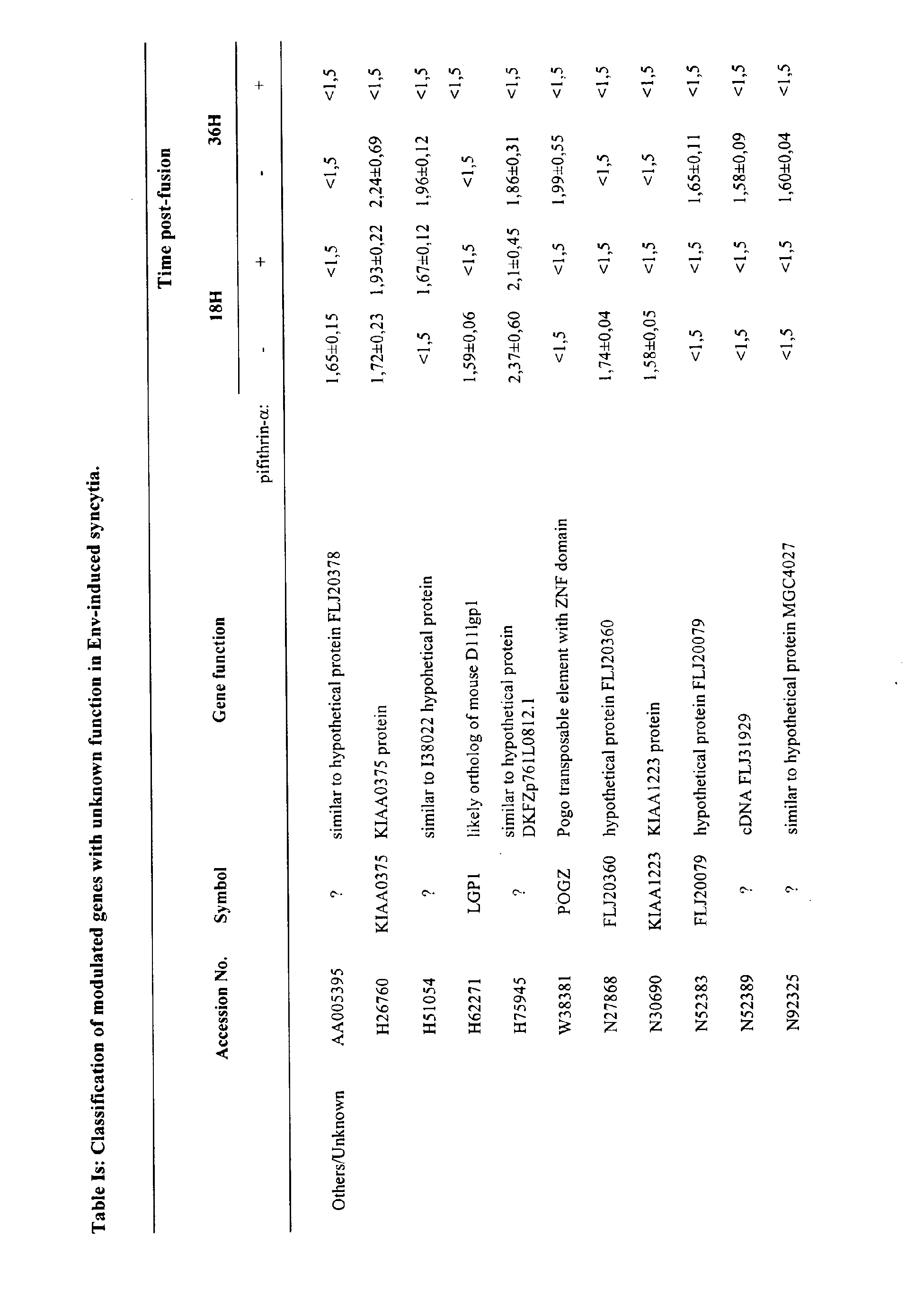

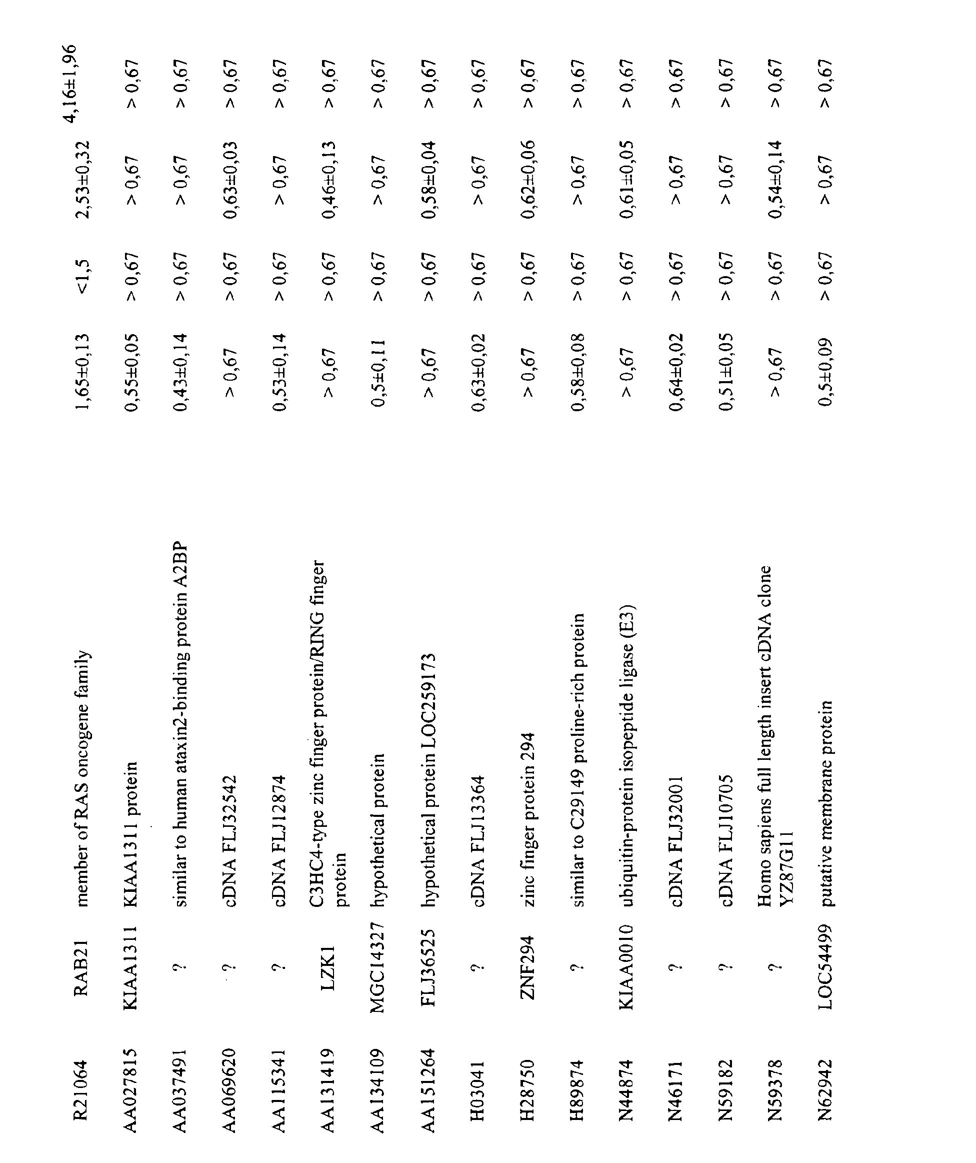

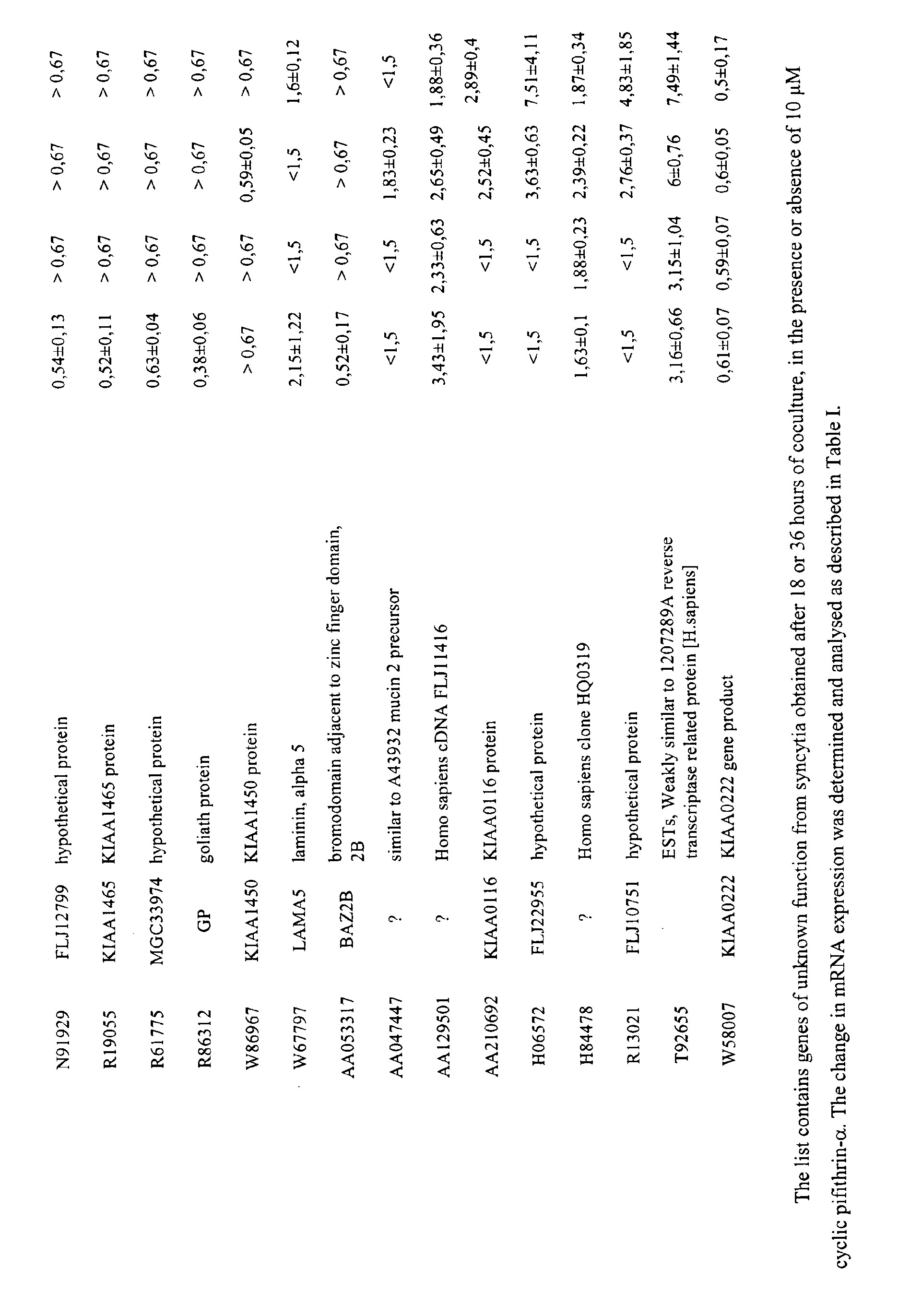

Based on the fact that the microarrays used in this study do not cover the entire transcriptome, we employed a p53-specific macroarray for the identification of genes specifically associated with syncytial apoptosis. Using this technique, we found that p53 itself, c-Jun (whose gene product, together with c-Fos, forms AP1), the pro-apoptotic gene PIG8 (also known as ei24), the pro-apoptotic Bcl-2 family member Bax, as well as the pro-apoptotic BH3-only Bcl-2 family member Puma were upregulated in syncytia by a factor = 2 (Figs. 5A, 5B). Quantitative RT-PCR confirmed the induction of Puma and Bax (and in particular the dominant Bax-α splice variant) transcripts (Fig. 6A), in line with the fact that syncytia overexpress Bax (5) and Puma (Fig. 6B) at the protein level.

### 4- Upregulation of Puma is required for syncytial apoptosis

Puma has recently been identified as a p53-inducible "BH3-only" protein that can activate the proteins Bax and Bak to adopt a pro-apoptotic conformation (with exposure of the N-terminus), to insert into the outer mitochondrial membrane, and to stimulate the release of cytochrome c leading to subsequent caspase activation and apoptosis (24). Accordingly, transfection with anti-sense oligonucleotides designed to down regulate the expression of Puma (see insert in Fig. 7B) inhibited the exposure of the N-termini of Bax and Bak (as detected with conformation-specific antibodies), the release of cytochrome c from syncytial mitochondria (as detected by immunofluorescence staining), and the apoptosis-associated chromatin condensation (Figs. 7A, 7B). Inhibition of NF-κB with IKSR (Fig. 7C) or SN50, as well as inhibition of p53 with cyclic pifithrin-α (Fig. 7D), all inhibited the activation of Bax and Bak. RNA interference of Bax or Bak, the two Puma receptors, significantly reduced syncytial apoptosis, as indicated by suppression of the nuclear chromatin condensation and the mitochondrial cytochrome c release (Fig. 7E). As a side observation, we found that RNA interference of Bak prevented the exposure of the N-terminus of Bax but not vice versa, suggesting a hitherto unsuspected hierarchy among these pro-apoptotic proteins. Altogether, the data suggest that Puma acts on Bax and/or Bak to kill syncytia in a p53-dependent fashion.

### 5- Induction of Puma in several models of HIV-1 and Env-induced apoptosis in vitro

When co-cultured with HeLa Env cells, U937 cells (which lack functional p53) (17), do not form heterokarya but rather undergo apoptosis after a transient cell-to-cell interaction (4). As show in Fig. 8A, apoptosis correlates with the induction of Puma, and the upregulation of Puma is not inhibited by cyclic pifithrin-αunderscoring that Puma can be induced in a p53-independent fashion. Similarly, different recombinant gp120 variants (MN and 93TH975 with preference for CXCR4, BaL and SF162 with preference for CCR5) induced cell death and Puma protein expression in U937 cells (which express both CXCR4 and CCR5), correlating with the induction of PUMA (Fig. 8B). Transfection of U937 cells with p53 accelerated the kinetics of gp120-induced Puma induction at early time points when gp120 alone still had no Puma-inducing effect (Fig. 8C). Thus p53-independent and -dependent pathway can cooperate to cause Puma induction. In (p53-sufficient) Jurkat cells (18), which only express CXCR4, only the R4-tropic gp120 variants induced apoptosis and Puma expression (Fig. 8D). This effect was strongly inhibited by cyclic pifithrin-α (Fig. 8E). Primary CD4+ lymphoblasts from healthy donors infected with HN-1LAI/IIIb also manifested the induction of Puma, at the protein level, well after the phosphorylation of p53 (Fig. 8F). CD4+ lymphoblasts infected with clinical HIV-1 isolates (12) manifested the induction of Puma, which could be detected in yet viable syncytia (Fig. 8G). Thus, Env and HIV-1 infection induce Puma expression in a variety of experimental systems.

### 6- Enhanced Puma expression in HIV-1-infected patients

Lymph nodes from untreated HIV-1 patients with high HIV-1 titers (>20,000 copies per ml) stained more positively for Puma than did biopsies from uninfected controls (Fig. 9A). Using immunocytochemical methods, Puma+ cells were found among all relevant (CD4+, CD8+ and CD14+) cells from HIV-1 infected donors with such high HIV-1 titers, with a preference for CD4+ and CD14+ cells (Fig. 9B). Puma expression levels among the PBMC correlated positively with HIV-1 titers (Fig. 9C). The enhanced Puma expression could also be detected by immunoblotting among circulating CD4+ cells from naive (non-treated) HIV-1 patients as compared to healthy controls or HAART-treated individuals (Fig. 9D). Moreover, longitudinal studies revealed that Puma levels were reduced to normal levels when patients were undergoing successful anti-retroviral therapy (Fig. 9C), in line with the well established fact that anti-retroviral therapy reduces the increased spontaneous apoptosis of circulating CD4+ lymphocytes from HIV-1 carriers (19). These data establish that productive HIV infection is associated with the overexpression of the apoptotic effector Puma.

### Concluding remarks.

These data strongly indicate that one of the dominant transcription factors activated in Env-elicited syncytia is p53. More than 80% of the genes whose expression level changed did so via a p53-dependent mechanism, as indicated by the fact that chemical p53 inhibition abolished this change. Inhibition of p53 prevented the expression of several pro-apoptotic genes and disabled the death program. p53 appears to be activated through at least two phosphorylation events, which are both detectable in HIV-1 carriers. One affects serine 15 and is mediated by mTOR, while the other affects serine 46. This latter phosphorylation event is well known to enhance the apoptogenic potential of p53 and to relay to mitochondrial apoptosis (20). Although the nature of the kinase acting on serine 46 (which is not mTOR, not shown in this example) remains to be determined, it appears clear that this phosphorylation event occurs downstream of the Cdkl-dependent karyogamy. Genetic or pharmacological inhibition of NF-κB prevented the cyclin B-dependent Cdkl activation, as well as the phosphorylation of p53 on both serine 15 and 46. Thus, the pro-apoptotic cooperation between NF-κB and p53 observed in this particular model is likely to be indirect, via a cell cycle-related effect of NF-κB. This would be in line with the fact that NF-κB is mostly viewed as an apoptosis-inhibitory transcription factor. NF-κB activation would be necessary for the advancement in cell cycle, which then enables p53-dependent transcription and apoptosis, yet would have no direct pro-apoptotic transcriptional effects. Indeed, there is no indication for the induction of anti-apoptotic NF-κB target genes in our system (Tables I and Is). In contrast, the p53-mediated expression of Puma appears to be direct, at least in Env-induced syncytia (Figs. 1-6). It is important to note that Puma expression is not under the exclusive control of p53, at least in transgenic mice (21), and that p53-independent pathways may contribute to the Env-elicited Puma expression (Figs. 8A, 8B, 8C) and actually cooperate with the p53-dependent ones (Fig. 8C). Thus, different pathways (p53 -dependent or -independent) may contribute to the induction of Puma, in vivo, in the HIV-1 infected patient. That at least part of this Puma-inducing effect involves p53 is suggested by the fact that p53 does manifest pro-apoptotic post-transcriptional modifications (in particular the phosphorylation of serine 46), which are detectable in resident and circulating immune cells from HTV-1 carriers.

A cornucopia of mechanisms contributes to the enhanced apoptotic decay of T lymphocytes in HIV-1 infection. Rather than viewing the virus-mediated and host-dependent contribution to lymphodepletion in an exclusive fashion, it is conceivable that pro-apoptotic mechanisms actually cooperate in the patient (1-3). As shown here, both soluble and membrane-bound Env can induce the expression of the pro-apoptotic protein Puma, a protein that acts on mitochondria to trigger apoptosis (24). Recently, it has also been shown that a mutation in Vpr that reduces its apoptogenic potential (R77Q) is found in ∼80% of long-term non-progressors, that is patients with high HIV-1 titers yet normal CD4 counts (and normal levels of spontaneous apoptosis). Vpr directly acts on mitochondria, on a molecular complex formed by Bax and the adenine nucleotide translocase, and can also elicit p53-dependent transcriptional effects (22). Although the *in vitro* systems that we characterized here were devoid of Vpr, these findings illustrate that the Env- and Vpr-elicited apoptotic pathways could cooperate in stimulating p53- and mitochondrion-mediated cell death. Such cooperative effects would be compatible with previous observations indicating a reduced expression of Bcl-2 in rarified CD4+ cells (23) and an enhanced level of Bax protein in those CD8+ cells that are undergoing apoptosis (24). Indeed Bcl-2 is known to be downregulated by p53 (25), while Bax is upregulated (26). The p53-induced imbalance in the precarious equilibrium of pro- and anti-apoptotic Bcl-2 relatives (which includes Puma and Bak, as shown here) would then favor the spontaneous or activation-induced apoptosis of T lymphocytes.

The co-culture of cells expressing the HIV-1 Envelope (Env) with cells expressing CD4 results into cell fusion, deregulated mitosis and subsequent cell death. This example I demonstrates that NF-κB, p53, and AP1 are activated in Env-elicited apoptosis. The NF-κB super-repressor had an anti-mitotic and anti-apoptotic effect and prevented the Env-elicited phosphorylation of p53 on serine 15 and 46, as well as the activation of AP1. Transfection with dominant-negative p53 abolished apoptosis and AP1 activation. Signs of NF-κB and p53 activation were also detected in lymph node biopsies from HIV-1 infected individuals. Microarrays revealed that most (85%) of the transcriptional effects of HIV-1 Env were blocked by the p53 inhibitor pifithrin-α. Macroarrays led to the identification of several Env-elicited, p53-dependent pro-apoptotic transcripts, in particular Puma, a pro-apoptotic "BH3-only" protein from the Bcl-2 family known to activate Bax/Bak. Down-modulation of Puma by anti-sense oligonucleotides, as well as RNA interference of Bax and Bak prevented Env-induced apoptosis. HIV-1 infected primary lymphoblasts upregulated Puma *in vitro.* Moreover, circulating CD4+ lymphocytes from untreated, HIV-1-infected donors contained enhanced amounts of Puma protein, and these elevated Puma levels dropped upon antiretroviral therapy. Altogether, these data indicate that NF-κB and p53 cooperate as the dominant pro-apoptotic transcription factors participating in HIV-1 infection.

### Example II: Essential role of p53 Phosphorylation by p38 MAPK in Apoptosis Induction by the HIV-1 Envelope

### A) Materials and Methods

### Cells and culture conditions for apoptosis induction

HeLa cells stably transfected with the Env gene of HIV-1 LAI (HeLa Env) and HeLa cells transfected with CD4 (HeLa CD4) were cocultured at a 1:1 ratio (29), in DMEM medium supplemented with 10% FCS, L-glutamine, in the absence or presence of SB203580 (100 nM), SB202190 (100 nM), SB202474 (100 nM), Caffein (500 µM), LY294002 (1 µM), PD98059 (5 µM), cyclic pifithrin-α (10 µM) obtained from Calbiochem.

### Plasmids, anti-sense constructs and transfection

Transfection with pcDNA3.1 vector only, or p38 DN plasmids (gift by Roger Davis), or HPLC-purified anti-sense HIPK2 phosphorothioate oligonucleotides (100 nM) and control were transfected with Lipofectamine (Invitrogen) into HeLa CD4 cells, 24 h before co-culture with HeLa Env cells. Transfection with pcDNA3.1 vector only or a p53-responsive enhanced green fluorescent (GFP) plasmid (kind gift from Klas Wiman) was performed 24 hours before coculture of HeLa CD4 and HeLa Env cells. The frequency of GFP-expressing cells was assessed by cytofluorometry, on a FACS Vantage (Becton Dickinson) equipped with a 100 µm nozzle, allowing for the analysis of relatively large cells. Cells were after counterstained with Hoechst 33324 (1 µM, 15 min), and the FACS gates were set on syncytia (that is cells with a DNA content >4n) (32).

### Immunofluorescence and cytofluorometry

Rabbit antisera specific for p38T180/Y182P, ATF-2T271P or the p53S46P (Cell Signaling Technology, MA, USA) was used on paraformaldehyde (4% w:v) and picric acid-fixed (0.19 % v:v) cells (34) and revealed with a goat anti-rabbit IgG conjugated to Alexa 568 (red) or Alexa 488 (green) fluorochromes from Molecular Probes. Cells were also stained for the detection of the cytochrome c (BD Pharmingen, USA) p53S15P (Cell Signaling Technology, MA, USA) revealed by anti-mouse IgG Alexa conjugates. Cells were counterstained with Hoechst 33324 which allows to distinguish karyogamy and apoptotic chromatin condensation (32).

### Immunoblots and Co-immunoprecipitation

Protein samples were prepared from HeLa Env and HeLa CD4 single cells mixed at a 1:1 ratio in Triton X-100 lysis buffer (single cells control) or from HeLa Env/CD4 syncytia. Aliquots of total protein extracts (400 µg) from lysates were incubated overnight at 4°C with 500 ng affinity-purified rabbit polyclonal antibodies specific for phospho-p38 MAP Kinase (T180/Y182P), followed by addition of 10 µl-packed proteinA/G agarose beads (1 h, 37°C; Santa Cruz Biotechnology, Inc.), vigourous washing of the pellet (10 min at 10,000 g, 3X) in PBS, 12 % SDS PAGE, and immunodetection with an p53-specific mAb.

### Patients' samples and in vitro infection with HIV-1

Axillary lymph node biopsies or peripheral blood samples were obtained from healthy and HIV-1-infected individuals (all males, mean age 36 years), who were naive for HAART with a plasma viral load >20,000 copies/ml; or were receiving HAART with a viral load <5,000 copies/ml. Plasma HIV-1 RNA levels were determined by the bDNA procedure (Versant HIV RNA 3.0) according to the manufacturer's instructions (Bayer, USA). None among the HIV-1+ patients received interferons, glucocorticoids, was positive for hepatitis B or C, or exhibited signs of autoimmunity. PBMCs were isolated by Ficoll/Hypaque (Amersham Pharmacia Biotech) centrifugation of heparinized blood from either healty donors and HIV-seropositive individuals and fixed with 4 % paraformaldehyde in PBS pH 7.2. Biopsies were fixed with 10 % formalin, dehydrated, and paraffin embedded. Deparaffinized tissue sections and PBMCs were subjected to immunocytochemistry with antibodies specific for p38T180/Y182P, p53S46P or PUMA and counterstained with hematoxylin. PBMC were treated with TRIzol (Invitrogen) to extract total proteins, followed by immunoblot analysis. Alternatively, freshly purified PBMC were stained with anti-CD4 antibody (FITC-labelled OKT3) and FACS-sorted, followed by protein extraction. Primary CD4+ lymphoblasts (10 x 10⁶) cells (30) were incubated with the HIV- 1_{LAI/IIIB} strain or different clinical HIV-1 isolates (12) (500 ng of p24) for 4 hours at 37°C. After washing out unabsorbed virus, cells (10⁶ cells/ml) were cultured in RPMI medium containing 20 % FCS and 10 U/ml interleukin-2 (IL-2).

### B) Results

### 1- Identification of the p53S46 kinase stimulated by HIV-1 Env as p38 MAPK

Syncytia formed by coculture of Env-transfected HeLa cells with a CD4/CXCR4-expressing HeLa cell line exhibited the phosphorylation of p53 on serine 46 (p53S46P), detectable in 30-40% of syncytia 36 hours after co-culture. Putative p53S46 kinases include ATM, HIP kinase-2 (21), and p38 MAPK (33). Inhibition of ATM with 500 mM caffeine (50) (Fig. 10A) as well as transfection with an anti-sense oligonucleotide designed to down-regulate the expression of HIP kinase-2 (32) (Fig. 10B) had no effect on the frequency of p53S46P+ syncytia (experiment system described in Example 2) and did not reduce the propensity of syncytia to undergo apoptosis (not shown). In strict contrast, SB203580 and SB202190, an inhibitor of p38 MAPK strongly reduced p53S46P. The inhibition of p53S46P was also observed with another p38 MAPK inhibitor, SB202190, but not with the inactive analog SB202474. Moreover, the inhibitors PD98059 and LY294002, which inhibit other tyrosine kinase than p38 MAPK, failed to affect p53S46P (Fig. 10C). Altogether, these data demonstrate that p38 is involved in the signal transduction cascade culminating in p53S46P.

### 2- Activation of p38 in Env-elicited syncytia and in HIV-1 infection

Using an antibody that recognizes phosphorylated p38 MAPK (p38T180P/Y182P), we detected activated p38 in HeLaEnv/CD4 co-cultures. p38T180P/Y182P was detectable early after syncytium formation, as soon as two cells had fused. At the pre-karyogamic stage p38T180P/Y182P was confined to the cytoplasm. During karyogamy, however, p38T180P/Y182P concentrated in the nucleus (Figs. 11A and 11C). The relocalization of p38T180P/Y182P correlated with the phosphorylation of one of its nuclear targets, ATF2, on Thr71 (ATF271P) (Fig. 11B). This phosphorylation of ATFZ was inhibited by SB203580 (which however had no effect on p38T180P/Y182P) (Fig. 11D), indicating that p38T180P/Y182P is indeed the kinase that phosphorylates ATF2 in karyogamic syncytia. Moreover, we found that p38T180P/Y182P co-immunoprecipitated with p53 (Fig. 11E), further underscoring that p38 MAPK is the kinase which phosphorylates p53 on serine 46 in karyogamic nuclei. Next, we investigated whether p38T180P/Y182P would be detectable in the context of HIV-1 infection. Indeed, p38T180P/Y182P was detectable in lymph node biopsies from HIV-1 treated individuals (Fig. 12A) as well as in HIV-1-infected cell cultures (Fig. 12B), but not in uninfected controls. Thus, p38 MAPK is activated in the context of HIV-1 infection.

### 3- Selective inhibition of p53S46P abolished p53-mediated transcription and apoptosis in Env-elicited syncytia

Specific inhibition of p38 MAPK by SB203580, used at a concentration of 100 nM, prevents the apoptogenic phosphorylation of p53 on serine 46 (see above). At this dose, SB203580 completely inhibited the expression of a p53-inducible GFP reporter gene construct transfected into the HeLa Env/CD4 co-culture system (Fig. 13A). However, SB203580 had no effect on the syncytial accumulation of cyclin B and thus did not block karyogamy. Moreover, SB203580 had no effect on the phosphorylatin of p53 on serine 15 (p53S15P) (Fig. 13B). The selective inhibition of p53S46P (without any effect on p53S 15P) (Fig. 4B) sufficed to block the transactivating function of p53, at least in this system. Accordingly, SB203580 prevented the mitochondrial release of cytochrome c and the apoptotic chromatin condensation normally occurring in Env-elicited syncytia (Fig. 13B). Similar results were obtained by transfection with a dominant-negative p38 mutant construct (Fig. 13C), thus confirming the results for the chemical p38 inhibitor SB203580. Altogether, these data underscore the critical apoptogenic role of p38 MAPK-mediated p53S46P for Env-induced apoptosis.

### 4- Highly active anti-retroviral therapy (HAART) reduces the activating phosphorylation of p38, the activating phosphorylation of p53 and the induction of PUMA

Circulating peripheral blood mononuclear cells (PBMC) from HIV-1 infected patients contain increased amounts of cells that stain positively with antibodies specific for p38T180P/Y182P (Figs. 14A and 14C), p53S46P (Fig. 14D) and PUMA (Fig. 14E). These percentages are reduced by HAART, to normal levels, among those patients which manifest a strong reduction in HIV-1 titers (Figs. 14A-14E) and increase again when HAART is interrupted for more than one month (Fig. 14C). This indicates that HIV-1 infection is linked to an activation of the p38/p53/PUMA axis (with an increase in the laboratory parameters, as quantified in Fig. 14), that successful therapeutic intervention reduces the activity of the pro-apoptotic p38/p53/PUMA signal transducing cascade to normal levels, and that therapeutic failure (as exemplified by interruption of HAART) causes a reactivation of the p38/p53/PUMA axis.

This example II demonstrates that the mitogen-activated protein kinase (MAPK) p38 plays a critical role in the induction of apoptosis by the envelope glycoprotein complex (Env) of human immundeficiency virus-1 (HIV-1). It is well known that the pro-apoptotic activity of the transcription factor p53 depends critically on the phosphorylation of serine 46 (p53S46P). An enhanced level of p53S46P can be detected in primary CD4+ lymphoblasts infected with HIV-1, in lymph node biopsies from HTV-1 carriers, and in cocultures of HeLa expressing the HIV-1 envelope (Env) with HeLa cells expressing CD4. In this latter model, cell death is the result of a sequential process involving cell fusion, abortive entry into mitosis with nuclear fusion (karyogamy), the phosphorylation of serine 15 (p53S15P), later p53S46P, and transcription of p53 target genes. Here, we show that pharmacological inhibitors of p38 mitogen-activated protein kinase (MAPK) reduced p53S46P, yet had no effect on karyogamy and p53S15P. Cytoplasmic p38 MAPK was found to undergo an activating phosphorylation (on threonin 180 and tyrosine 182) as early as at the two-cell stage of syncytium formation. Phosphorylated p38 MAPK translocated to the nucleus when karyogamy occurred, correlating with the phosphorylation of the p38 MAPK target ATF2. Inhibition of p38 MAPK suppressed the expression of p53-inducible genes, the release of cytochrome c from mitochondria, and consequent apoptosis. Importantly, we found that p38 MAPK phosphorylation also occurred in lymph nodes and circulating white blood cells from HIV-1 carriers and that this enhanced p38 MAPK phosphorylation was reduced upon successful treatment with HAART (highly active anti-retroviral therapy). These findings indicate that p38 MAPK-mediated p53 phosphorylation constitutes a critical step of HIV-1-induced apoptosis.

### References

1. Pantaleo, G. et al., 1995, Nat Med. 1:118-120.
2. Badley, A.D. et al., 2000, Blood 96:2951-2964.
3. Gougeon, M., 2003, Nat Rev Immunol, 3:392-404.
4. Ferri, K.F. et al, 2000, J. Exp. Med.,192:1081-1092.
5. Castedo, M. et al., 2001, J. Exp. Med., 194:1097-1110.
6. Genini, D. et al., 2001, FASEB J. 15:5-6.
7. Castedo, M. et al., 2002, EMBO J., 21:4070-4080.
8. Piedimonte, G. et al., 1999, Aids, 13:1159-1164.
9. Moretti, S. et al., 2000 Clin. Exp. Immunol., 122:364-373.
10. van den Heuvel, S. et al., 1993, Science, 262:2050-2054.
11. Daugas, E. et al., 2000, FASEB J., 14:729-739.
12. Bartolini, B. et al., 2000, Exp. Cell Sci,. 261:119-126.
13. Kaltschmidt, B., 1999, Oncogene, 18:3213-3225.
14. Hollstein, M. et al., 1991, Science, 253:49-52.
15. Karin, M. et al., 2002, Nat Rev Cancer, 2:301-310.
16. Nakano, K. et al., 2001, Mol. Cell 7:683-694.
17. Stiewe, T. et al., 2000, Nat Genet., 26:464-469.
18. Gualberto, A. et al., 1998, J. Biol. Chem., 273:7088-7093.
19. Autran, B. et al., 1997, Science 277:112-116.
20. Oda, K. et al., 2000, Cell, 102:849-862.
21. Villunger, A. et al., 2003, Science, 302:1036-1038.
22. Chowdhury, I.H. et al., 2003, Virology, 305:371-377.
23. David, D. et al., 2002, AIDS, 16:1093-1101.
24. Ledru, E. et al., 1998, J. Immunol. 160:3194-3206.
25. Miyashita, T. et al., 1994, Cancer Res., 54:3131-3135.
26. Miyashita, T., et al., 1995, Cell, 80:293-299.
27. Komarov, P. G. et al., 1999, Science, 285, 1733-1737.
28. Corbeil, J. et al., 2001, Genome Res., 11, 1198-204.
29. Mihara, M. et al., 2003, Mol. Cell, 11, 577-90.
30. Vousden, K. H. et al. 2002, Nat Rev Cancer, 2, 594-604.
31. Saito, S. et al., 2002, J Biol Chem, 277, 12491-4.
32. Hofmann, T. G. et al., 2002, Nat Cell Biol, 4, 1-10.
33. Bulavin, D. V. et al., 1999, EMBO, J. 18, 6845-54.
34. Sarkaria, J. N. et al., 1999, Cancer Res., 59, 4375-4382.
35. Fire, A. et al., 1998, Nature, 391:806-811.
36. Elbashir, S.M. et al., 2001, Nature, 411:494-498.
37. Caplen, N. J.et al., 2001, Proc Natl Acad Sci USA, 98: 9742-9747.
38. Hutvagner, G. et al., 2001, Science 293: 834-838.

## Claims

1. A method for the *in vitro* screening of anti-HIV compounds comprising the following steps of:
(a) forming a mixture of a compound to be tested with a sample containing HIV infected circulating white blood cells;
(b) measuring of the Puma expression level in said HIV infected circulating white blood cells,
wherein a compound that decreases the Puma expression level in said circulating white blood cells is selected as an anti-HIV compound.

2. A method for the *in vitro* screening of anti-HIV compounds according to claim 1,
wherein the step (b) further comprises a step of measuring of the phosphorylated p38 MAP kinase protein level and/or the phosphorylated p53 protein level in said HIV infected circulating white blood cells, and wherein a compound that decreases in said circulating white blood cells:
- the Puma expression level; and
- the phosphorylated p38 MAP kinase protein level and/or the phosphorylated p53 protein level, is selected as an anti-HIV compound.

3. A method for predicting the progression of HIV related diseases in a HIV infected patient, said method comprising the following step:
(a) from a sample of said patient containing HIV infected circulating white blood cells, measuring of the Puma expression level in said HIV infected circulating white blood cells.

4. A method for predicting *in vitro* the progression of HIV-related diseases in an HIV infected patient according to claim 3, wherein an increase of the Puma expression level in said HIV infected circulating white blood cells, compared with reference circulating white blood cells of a HIV non infected patient is significant of HIV induced circulating white blood cells apoptosis in said infected patient.

5. A method for predicting the progression of HIV related diseases according to claim 3 or 4, wherein in claim 3, the step (a) further comprises a step of measuring the phosphorylated p38 MAP kinase protein level and/or the phosphorylated p53 protein level and wherein, in claim 4, an increase in said circulating white blood cells of:
- the Puma expression level; and
- the phosphorylated p38 MAP kinase protein level and/or the phosphorylated p53 protein level, compared with reference circulating white blood cells of a HIV non infected patient, is significant of HIV induced circulating white blood cells apoptosis in said infected patient.

6. A method for determining *in vitro* the efficiency of an anti-HIV treatment for a HIV infected and treated patient, said method comprising the following step:
(a) obtaining a sample of said anti-HIV treated patient, said sample containing circulating white blood cells; and
(b) measuring of the Puma expression level in said circulating white blood cells,
wherein the efficiency of said anti-HIV treatment is correlated with the decrease of the amount of the Puma expression level measured in said circulating white blood cells.

7. A method for determining *in vitro* the efficiency of an anti-HIV treatment susceptible to be administrated to a HIV infected patient, said method comprising the following step:
(a) forming a mixture of the anti-HIV treatment compounds susceptible to be administrated with a sample containing HIV infected circulating white blood cells from a sample of said non-treated patient containing HIV infected circulating white blood cells; and
(b) measuring of the Puma expression level in said circulating white blood cells,
wherein the efficiency of said anti-HIV treatment is correlated with the decrease of the amount of the Puma expression level measured in said circulating white blood cells.

8. A method for determining *in vitro* the efficiency of an anti-HIV treatment according to claim 6 or 7, wherein the step (b) further comprises a step of measuring of the phosphorylated p38 MAP kinase protein level and/or the phosphorylated p53 protein level, and
wherein the efficiency of said anti-HIV treatment is correlated with the decrease measured in said circulating white blood cells of:
- the amount of Puma expression level; and
- the amount of phosphorylated p38 MAP kinase protein level and/or the phosphorylated p53 protein level.

9. A method for diagnosing *in vitro* the HIV infection of a patient susceptible to be infected with HIV, said method comprising the following step of:
(a) from a sample of said patient susceptible to be infected with HIV, said sample containing circulating white blood cells, measuring of the Puma expression level in said circulating white blood cells,
wherein the increase of the amount of the Puma expression level measured in said circulating white blood cells, is significant with a HIV infection of said patient.

10. A method for diagnosing *in vitro* the HIV infection according to claim 9, said method further comprising in step (a) the measuring of the phosphorylated p38 MAP kinase protein level and/or the phosphorylated p53 protein level in said circulating white blood cells,
wherein the increase measured in said circulating white blood cells of:
- the amount of Puma expression level; and
- the amount of phosphorylated p38 MAP kinase protein level and/or the phosphorylated p53 protein level,
is significant with a HIV infection of said patient.

11. The method of claims 1 to 10, wherein the expression level of Puma which is measured in step (a) or (b) is the measuring of the Puma protein level.

12. The method of claims 1 to 10, wherein the expression level of Puma which is measured in step (a) or (b) is the measuring the Puma transcripts level.

13. The method of claim 2, 5, 8 or 10, wherein the measuring of the phosphorylated p53 protein level in step (a) or (b) is the measuring of the phosphorylated p53S46P protein level.

14. The method of claims 2, 5, 8, 10 or 13, wherein the measuring of the phosphorylated p38 MAP kinase protein level in step (a) or (b) is the measuring of the phosphorylated p38T180P or/and p38Y182P protein level.

15. The method of claims 1 to 14, wherein the measuring of said Puma protein level, and, optionally, said phosphorylated p38 MAP kinase protein level and/or phosphorylated p53 protein level is carried out by specific antibodies capable of specifically recognizing said proteins.

16. The method of claims 1 to 15, wherein HIV is HIV-1.

17. Kit for the *in vitro* diagnostic of HIV infection in a patient, **characterized in that** it comprises:
(a) anti-human Puma protein antibodies or a set or primers capable of amplifying the human Puma mRNA or cDNA.

18. Kit according to claim 17, wherein said kit further comprises:
(b) anti-human phosphorylated p38 MAP kinase antibodies; and/or
(c) antibodies anti-human phosphorylated p53 protein antibodies.

19. Kit according to claim 18, **characterized in that** anti-human phosphorylated p38 MAP kinase antibodies are anti-human phosphorylated p38T180P antibodies or/and anti-human phosphorylated p38Y182P antibodies.

20. Kit according to claims 18 and 19, **characterized in that** anti-human phosphorylated p53 protein antibodies are anti-human phosphorylated p53S46P antibodies.

21. Kit according to claims 17 to 20, **characterized in that** HIV is HIV-1.
